# EUROPEAN PATENT APPLICATION

(11) **EP 2 527 008 A2**
(43) Date of publication of application: **28.11.2012**
(21) Application number: 12178655.2
(22) Date of filing: 28.04.2008
(51) Int. Cl.: A61P 35/00, A61K 31/785, A61K 38/02, A61K 47/48, C07K 7/02, A61K 38/12, A61K 45/06

(54) **Anticancerous polymeric agents**

(30) Priority: 30.04.2007 US 924088 P
(62) Divisional of application: 08738266.9
(71) Applicant: Technion Research & Development Foundation Ltd., 32000 Haifa (IL)
(72) Inventor: Mor, Amram, 36700 Nesher (IL); Held-Kuznetsov, Viktoria, 36821 Nesher (IL)
(74) Representative: Becker Kurig Straus

(57) **Abstract**

Methods and pharmaceutical compositions for treating cancer, particularly MDR cancer, which utilize polymeric compounds that are composed of a plurality of amino acid residues and one or more hydrophobic moieties are disclosed.

## Description

### FIELD AND BACKGROUND OF THE INVENTION

The present invention relates to anti-cancer polymeric agents and, more particularly, to the use of polymers which are designed to exert anticancer activity while being stable, non-toxic and avoiding development of multiple drug resistance. The present invention further relates to pharmaceutical compositions containing such polymers and to methods of treating cancer utilizing same.

### Cancer:

Cancer is a genetic disease in which mutations violate cell growth and survival pathways. Essentially abnormal tissue growth (neoplasm) develops through a process whereby cancer begins in a single cell and passes its malignant potential to subsequent generations of cells. A carcinogenic event is usually operated by some external disruptive factors, such as viruses, radiation (such as sunlight, x-rays and radioactive sources which emit energy and subatomic particles) and chemical carcinogens, mutagens or teratogens. Mammalian cells have multiple safeguards to protect them against the potentially lethal effects of cancer gene mutations, but when several genes are defective, an invasive cancer develops. Human cancers originate from mutations that usually occur in somatic tissues; however, hereditary forms of cancer exist in which individuals are heterozygous for a germline mutation.

The mutations target three types of genes (cancer genes), namely tumor suppressor genes, oncogenes, and stability genes. Loss-of-function mutations in tumor suppressors and gain-of-function mutations in oncogenes lead to cancer, while loss-of-function mutations in stability genes increase the rates of mutation of tumor suppressors and oncogenes. All cancer mutations operate similarly at the physiologic level: they drive the carcinogenic process by increasing tumor cell number through the stimulation of cell birth or the inhibition of cell-cycle arrest or cell death. The increase is usually caused by facilitating the provision of nutrients through enhanced angiogenesis, by activating genes that drive the cell cycle or by inhibiting normal apoptotic processes.

### Cancer treatment and chemotherapy:

The most common types of cancer treatment are surgery, radiotherapy and chemotherapy. Radiotherapy is usually used alone or in combination with surgery and/or chemotherapy. Other types of treatments include hormone therapy that is used in combination with surgery and/or chemotherapy for treatment of, for example, androgen-dependent prostate cancer or estrogen-dependent breast cancer.

Cryosurgery uses cold liquid nitrogen or gas argon to destroy abnormal tissue. Relatively new additions to the family of cancer treatments include biological therapy and angiogenesis inhibitors. Biological therapy is based on the stimulation of the body's own immune system, either directly or indirectly, to fight off cancer or to diminish side effects caused by other treatments.

To date, chemotherapy remains the most common and most frequently used in cancer treatment, alone or in combination with other therapies. Currently available anticancer chemotherapies act by affecting specific molecular targets in proliferating cancer cells, leading to inhibition of essential intracellular processes such as DNA transcription, synthesis and replication.

Unfortunately anticancerous drugs are highly toxic, as they are designed to kill mammalian cells, and are therefore harmful also to normal proliferating cells resulting in debilitating and even lethal side effects. Some of these adverse effects are gastrointestinal toxicity, nausea, vomiting, and diarrhea when the epithelial lining of the intestine is affected. Other side effects include alopecia, when the hair follicles are attacked, bone marrow suppression and neutropenia due to toxicity of hematopoietic precursors. Therefore the effectiveness of currently used anticancerous drugs is dose-limited due to their toxicity to normal rapidly growing cells.

One of the contemporary approaches in the fight against cancer is engineering of molecular targeted drugs that permeate cancer cells and specifically modulate activity of molecules that belong to signal-transduction pathways. These targets include products of frequently mutated oncogenes, such as k-Ras and other proteins that belong to tyrosine kinase signal transduction pathways. For example, Imatinib (Gleevec®), is the first such drug, approved for treatment of chronic myelogenous leukemia (CML). Imatinib blocks the activity of non-receptor tyrosine kinase BCR-Abl oncogene, present in 95 % of patients with CML. Imatinib was found to be effective in the treatment of CML and certain tumors of the digestive tract. Nevertheless, as others, this new compound is not completely specific to its target; therefore side effects emerge, including severe congestive cardiac failure, pulmonary tuberculosis, liver toxicity, sweet syndrome (acute febrile neutrophilic dermatosis), leukocytosis, dermal edemas, nausea, rash and musculoskeletal pain.

Angiogenesis inhibitors are currently investigated for their use in cancer treatment and to date, one anti-angiogenetic drug, Bevacizumab (Avastin®), was approved for the treatment of solid tumors in combination with standard chemotherapy. However, as in all chemotherapeutic drugs, Bevacizumab causes a number of adverse side effects such as hypertension, blood clots, neutropenia, neuropathy, proteinuria and bowel perforation.

### Multidrug resistance (MDR):

Treatment of cancer is becoming even more complicated, since on top of the many factors that cause tumor formation and the multiple adverse side effects associated with currently available anticancerous agents, there are a myriad of mechanisms by which cells become resistant to unspecific drugs.

Mechanisms of drug resistance include prevention from entering the cells, pumping the drug out of the cells, enzymatic inactivation, prevention of drug activity by mutation or altered expression of the target, and inhibition of biochemical pathways by mutations in oncogenes, tumor-suppressor genes or stability genes.

Many of the most prevalent forms of human cancer resist effective chemotherapeutic intervention. Some tumor populations, especially adrenal, colon, jejunal, kidney and liver carcinomas, appear to have drug-resistant cells at the outset of treatment [Barrows, L. R., "Antineoplastic and Immunoactive Drugs", Chapter 75, pp 1236-1262, in: Remington: The Science and Practice of Pharmacy, Mack Publishing Co. Easton, Pa., 1995]. In other cases, a resistance-conferring genetic change occurs during treatment; the resistant daughter cells then proliferate in the environment of the drug. Whatever the cause, resistance often terminates the usefulness of an anticancerous drug, and the emergence of multidrug resistance (MDR) sadly lead to therapeutic failure in many cancer patients [Liscovitch, M. and Lavie, Y., IDrugs, 2002, 5(4), 349-55].

Many researches have been conducted in order to elucidate the mechanism behind the development of MDR cancer cells. One of the most recognized mechanisms involves the ABC (ATP Binding Cassette) transporter proteins. These proteins are capable of coupling the energy of ATP binding and hydrolysis, so as to transport substrates across a cell membrane. The normal physiological role of these proteins is detoxification and clearance by active secretion of intracellular xenobiotic and other undesired substances out of the cell. Thus, in order to ultimately perform their normal physiological role, nature has designed these proteins capable of extruding a wide scope of molecules.

Due to their recognized activity in multidrug resistance (MDR) in tumor chemotherapy these transporter proteins are widely termed in the art as "MDR extrusion pumps".

The lowered efficacy of chemotherapy is linked to the fact that MDR extrusion pumps are over-expressed in cancer cells, as compared to their expression level in normal cells, and are responsible for pumping chemotherapeutic drugs out of the cell, which reduces the levels of intracellular drug below lethal thresholds regardless of the of nature of the cancer cell and/or the drug.

This mechanism of resistance may account for de novo resistance in common tumors, such as colon cancer and renal cancer, and for acquired resistance, as observed in common hematologic tumors such as acute nonlymphocytic leukemia and malignant lymphomas.

Both the resistance to conventional drugs monotherapy and the toxicity of currently use chemotherapeutic agents, support the rationale for combination drug therapy and the use of agents that can fight MDR. Compounds capable of inhibiting MDR extrusion pumps are known in the art as chemosensitizers or chemosensitizing agents. Combination of drugs with different modes of action may protect normal cells against chemotoxicity [Carvajal, D. et al., Cancer Res., 2005, 65, 1918-1924] or facilitate chemotherapy action on resistant tumors [Molnar, J. et al., Curr. Pharm. Des, 2006, 12, 287-311].

### Antimicrobial peptides:

Antimicrobial proteins and peptides (AMPs) is a vast family of compounds currently under study which are typically characterized by a flexible structure, an amphiphatic character and a net positive charge. AMPs are derived from animal sources and constitute a large and diverse family of peptides. In the past 25 years, over 700 AMPs derived from various sources, from unicellular organisms to mammalians and including humans, have been identified [Gordon, Y. J., E. G. Romanowski, et al. (2005), Curr Eye Res 30(7): 505-15; Stallmann, H. P., C. Faber, et al. (2006), Injury 37 Suppl 2: S34-40; and Yedery, R. D. and K. V. Reddy (2005), Eur J Contracept Reprod Health Care 10(1): 32-42].

AMPs are well recognized as having an important role in the innate host defense. They display a large heterogeneity in primary and secondary structures but share common features such as amphiphatic character and net positive charge. These features appear to form the basis for their cytolytic function. The molecular mechanism of action of AMPs appears to differ among different families of these molecules. Ample data indicate that AMPs cause cells death by destabilizing the ordered structure of the cell membranes, although the detailed mechanism has not been fully understood yet. In addition, due to the variability of cell membrane ultrastructures, a given peptide may act via different mechanisms in distinct membrane environments. It is assumed that disturbance in membrane structure leads to leakage of small solutes (for example K⁺, amino acids and ATP) rapidly depleting the proton motive force, starving cells of energy and causing cessation of certain biosynthetic processes [Sahl, H. G. and Bierbaum, G. (1998), Annu. Rev. Microbiol. 52, 41-79].

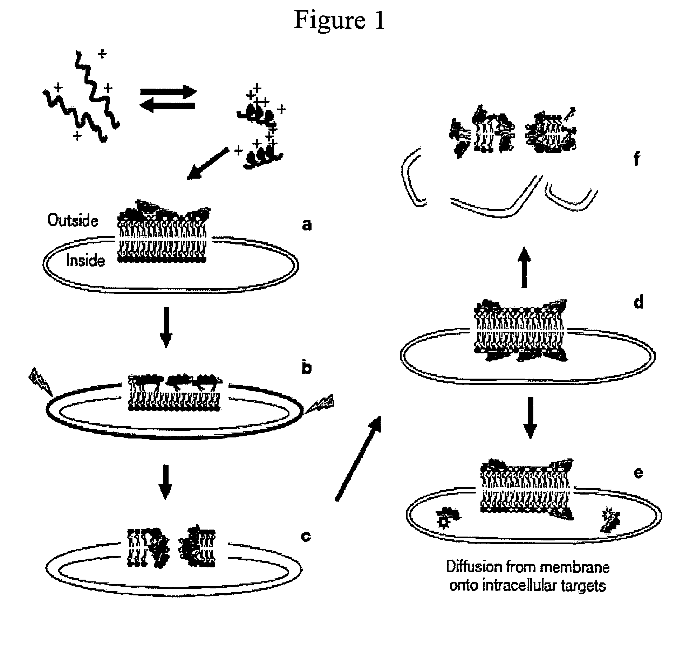

Figure 1 illustrates schematically a summary of the common features which are shared by the various proposed mechanism of action of AMPs, which were extracted from activity studies of individual peptides against artificial membranes. Initially, it is assumed that the peptides bind to the negatively charged phospholipids head groups on the target cell membrane through electrostatic interactions, followed by peptide insertion into the hydrophobic environment of the membrane, which is facilitated by adoption of amphiphatic conformation with the peptide axes oriented parallel to the plane of membrane (Figure 1a). When the bound peptides attain a threshold concentration, they permeate the outer leaflet of the bilayer, altering the order of phospholipids, and interacting with the cytoplasmic leaflet. This leads to thinning off and expanding of the outer leaflet relative to the cytoplasmic leaflet and resulting in membranal strain (Figure 1b). The strain, and possibly the strong trans-negative potential of the cell, facilitates cationic peptide transitions in orientation on the membrane and entry into the membrane core, forming a transient pores or channel. The peptides are inserted into the bilayer perpendicular to the plane of membrane and their hydrophobic side-chains interact with the lipid acyl chains (Figure 1c). Next, the peptides may insert and bind to the cytoplasmic leaflet of the membrane, inducing increased rate of conformational changes of phospholipids (Figure 1d). Finally, the peptides may translocate into the cytoplasm and alter normal functions of intracellular targets, inhibiting vital processes (Figure 1e). Otherwise, peptides may induce membrane depolarization and complete disordering of phospholipids' compositional specificity that leads to membrane breakup (Figure If). This model of the molecular mechanism of action of AMPs is helpful in defining chemical characteristics which are crucial for AMP activity, although their relevance in resolving the question of how peptides kill specific pathogens or cells is not fully clear.

### Antimicrobial peptides as anticancerous agents:

Some reports have suggested AMPs as anticancerous agents, and demonstrated that in addition to their potent antimicrobial activity, AMPs exhibit selective cytotoxicity against a broad spectrum of cancer cells [Papo, N. et al., J. Biol. Chem., 2003, 278, 21018-21023; Cruciani, R. A. et al., Proc. Natl. Acad. Sci. U. S. A, 1991, 88, 3792-3796; Chen, H. M. et al., Biochim. Biophys. Acta, 1997, 1336, 171-179; Yang, N. et al., J. Pept. Res., 2002, 60, 187-197; and Chen, J. et al., Cancer Res., 2005, 65, 4614-4622;57-64]. The well established biological activities of AMPs both *in vitro* and *in vivo* suggest that such peptides may have future value as anticancer agents either alone or in combination with conventional therapies.

Examples of AMPs antitumor activities include magainin analogues that were shown to inhibit carcinoma cells in vitro and proved to be as effective as doxorubicin in inhibiting an advanced stage of the murine ovarian tumor when administered by intraperitoneal injection [Baker, M. A. et al., Cancer Res., 1993, 53, 3052-3057]. Another study demonstrated that intratumoral injections of bovine lactoferricin (LfcinB) slowed the growth of murine fibrosarcoma cells in mice, and in addition, subcutaneous administration of LfcinB one day after intravenous tumor inoculation results in a significant inhibition of lung and liver metastasis of murine lymphoma cells as well as lung metastasis of murine melanoma cells [Yoo, Y. C. et al., Jpn. J. Cancer Res., 1997, 88, 184-190]. Moreover, diastereomers of synthetic antimicrobial peptides showed activity against melanoma, lung and prostate carcinoma cells in culture, and also inhibited lung, prostate and breast human tumors and eliminated a multiple metastases in mouse model without causing damage to the mice's vital organs [Papo, N. et al., Biochemistry, 2003, 42, 9346-9354, Cancer Res., 2004, 64, 5779-5786 and Cancer Res., 2006, 66, 5371-5378].

The higher susceptibility of cancer cells to the cytolytic activity of AMPs, as compared to that of normal cells, can be attributed to specific factors that are implicated in neoplasia, such as heightened concentration of negatively charged cellular components of the cancer cells. Indeed, as depicted above, the plasma membrane of cancer cells seems to have a higher content of negatively charged molecules such as phosphatidylserine [Utsugi, T. et al., 1991, Cancer Res. 51, 3062-3066], sialic acid [Miyagi, T. et al., Glycoconj. J., 2004, 20, 189-198], hyaluronan or closely related glycosaminoglycans [Bullard, K. M. et al., Int. J. Cancer, 2003, 107, 739-746] compared to normal cells. Moreover, the relatively higher number of microvilli on tumorigenic cells may consequently increase the surface area of the tumorigenic cell membranes and enables binding of a larger amount of the peptides [Chan, S. C. et al., Anticancer Res., 1998, 18, 4467-4474].

Mitochondrial membrane was proposed as another possible target of AMPs. The rational for this proposal is based on the idea that eukaryotic mitochondria have evolved from prokaryotic cells and possess negatively charged membrane components as well as a highly negative transmembrane potential [Gray, M. W. et al., Science, 1999, 283, 1476-1481]. Hence, a cecropin-melittin hybrid peptide was shown to permeabilize the inner membrane of rat liver mitochondria, allowing the movement of both charged and non-charged analytes [az-Achirica, P. et al., Eur. J Biochem., 1994, 224, 257-263]. In intact cells, the membrane-active peptide can induce the permeation, depolarization and swelling of mitochondria, which leads to apoptosis. For example, a synthetic membrane-active antimicrobial peptide fused to a tumor blood vessel homing domain exhibited antitumor activity by targeting mitochondria and triggering apoptosis of angiogenic endothelial cells [Ellerby, H. M. et al., Nat. Med., 1999, 5, 1032-1038], and LfcinB selectively induced apoptosis by triggering the mitochondrial pathway in human leukemia and carcinoma cell lines [Mader, J. S. et al., Mol. Cancer Ther., 2005, 4, 612-624].

In addition to a membrane-linked mode of cytotoxic action, AMPs may affect intracellular targets [Papo, N. and Shai, Y., Cell Mol. Life Sci., 2005, 62, 784-790]. Intracellular activities of AMPs may include inhibiting acutely infected cell-associated production of HIV-1 by suppressing HIV-1 gene expression [Wachinger, M. et al., J. Gen. Viol, 1998, 79 (Pt 4), 731-740] and DNA synthesis in 3T3 fibroblast cells, inhibition correlated with intracellular uptake of the proline-rich AMP [Tomasinsig, L. et al., J. Biol. Chem., 2006, 281, 383-391]. AMPs can target tumor suppressor or oncogene activities by direct or indirect effect on participants of their signal transduction pathways. For instance, mellitin was reported to act specifically against cells in cultures that express high level of the Ras oncogene by preferential hyperactivation of PLA2, resulting in selective destruction of these cells [Sharma, S. V., Oncogene, 1992, 7, 193-201]. Similarly, tachyplesin was shown to inhibit proliferation of human hepatocellular carcinoma cells by the upregulation of tumor-suppressor protein p21 and downregulation of the oncogene protein c-myc [Ouyang, G. L. et al., World J. Gastroenterol., 1992, 8, 1053-1058].

### Antimicrobial peptides and multidrug resistance:

Although probably based on different mechanisms, both cancer treatment and treatment against pathogenic microorganisms share the problem of the occurrence of drug resistance as a result of treatment with one specific drug. The known therapeutic traits of antimicrobial peptides formed the basis for anticancer studies involving drug-resistant cancer cells [Kim, S.S. et al., 2003, Peptides, 24(7): 945-53]. AMPs were suggested as effective anticancerous agents particularly in cases where the cancer cells become resistant to conventional chemotherapy [Papo, N. and Shai, Y., Biochemistry, 2003, 42(31), 9346-9354; Mader, J.S. and Hoskin, D.W., Expert Opinion on Investigational Drugs, 2006, 15(8), 933-946(14)].

The proposed mechanism of action of AMPs is consistent with the hypothesis that antimicrobial activity is not mediated by interaction with a chiral center [Krugliak, M., et al., Antimicrob Agents Chemother, 2000, 44(9), 2442-2451] and may thus significantly prevent multidrug-resistance by circumventing many of the mechanisms known to induce resistance.

Pore-forming proteins and granzyme B (exogenous serine proteases that are released by cytoplasmic granules within cytotoxic T cells and natural killer cells) were shown to induce apoptosis in cells induced to over-express a P-glycoprotein [Johnstone, R. W. et al., Blood, 1999, 93, 1075-1085]. Most membrane-active AMPs bind rapidly to the plasma membrane of cancer cells and disrupt it, probably, in a similar way to that of the pore-forming proteins and granzymes, and this non-specific mechanism of cell killing seem to overcome the inherent resistance of cancer cells. Indeed, the high potency of magainin analogues against six small cell lung cancer (SCLC) cell lines was shown not to be affected by the MDR1 gene [Ohsaki, Y. et al., Cancer Res., 1992, 52, 3534-3538], and the cell death was induced by forming ion channels in a lipid bilayer membrane [Cruciani, R. A. et al., Eur J Pharmacol., 1992, 226, 287-296]. Magainin 2 was also found to kill BRO melanoma cells transfected with the MDR1 gene with the similar potency as induced death in the parent BRO cells [Lincke, C. R. et al., Cancer Res., 1990, 50, 1779-1785]. These peptides probably act through the above-mentioned non specific mechanism of action of AMPs against target membranes. Similarly, diastereomers of synthetic antimicrobial peptides were shown to depolarize the transmembrane potential of cancer cells at the same rate measured in minutes and at concentrations suitable for anticancer activity.

The mode of action of antimicrobial peptides triggered studies of their synergistic effect with conventional chemotherapeutics. Magainin analogues were shown to enhance antitumor effects when combined with standard chemotherapeutic agents such as cisplatin, etoposide and doxorubicin [Ohsaki, Y. et al., Cancer Res., 1992, 52, 3534-3538]. Cecropin could act synergistically with conventional chemotherapeutics such as 5-fluorouracil and cytarabine against acute lymphoblastic leukemia cells, and diastereomer of a synthetic peptide act synergistically with doxorubicin toward androgen-independent and androgen-dependent prostate carcinoma cells.

### The shortcomings of AMPs as pharmaceutical agents:

While the potential of AMPs as new therapeutic agents is well recognized, as well as their anticancerous activity, the use of the presently known AMPs is limited by lack of adequate specificity, and optional systemic toxicity [House of Lords, Science and Technology 7th Report: Resistance to antibiotics and other antimicrobial agents. HL Paper 81-II, session, 1997-98; and Alan, A. R. et al. (2004), Plant Cell Rep. 22, 388-396].

Most AMPs are sensitive polypeptides and have often a short circulatory half-life *in vivo,* and therefore the pharmaceutical uses thereof are critically limited by their *in vivo* and *ex vivo* instability and by their poor pharmacokinetics. This is particularly valid for non-glycosylated polypeptides of a molecular mass less than 50 kDa.

The short lifetime of proteins *in vivo* is attributed to several factors, including glomerular filtration in the kidney, and proteolysis in the stomach, bloodstream and liver. Moreover, AMPs are typically characterized by poor absorption after oral ingestion due to the lack of specific transport systems and other chemical and physical criteria such as aqueous solubility, structural flexibility and size, and net molecular charge. Polypeptides are easily degraded in oxidative and acidic environments and therefore typically require intravenous administration (so as to avoid, e.g., degradation in the gastrointestinal tract).

Considering that AMPs are not absorbed orally, prolonged maintenance of these therapeutically active polypeptides in the circulation is still a considerable challenge of great clinical importance, since polypeptides are further broken down in the blood system and liver by proteolytic enzymes and are rapidly cleared from the circulation, and further have the tendency to evoke an immunological response particularly when their sequence is not recognized by the host's immune system.

In addition, AMPs are heat and humidity sensitive and therefore their maintenance requires costly care, complex and inconvenient modes of administration, and high-cost of production and maintenance. The above disadvantages impede the use of proteins as efficient drugs and stimulate the quest for means to alter some of the characteristics of proteins so as to bestow robustness and stability thereto.

### Overcoming AMPs shortcomings:

Peptidomimetic compounds are modified polypeptides which are designed to have a superior stability, both *in vivo* and ex *vivo,* and yet at least the same receptor affinity, as compared with their parent peptides. In order to design efficacious peptidomimetics, a careful attention must be drawn to the characteristics which are responsible for their interaction with the intended target is therefore required.

U.S. Patent Application Nos. 11/234,183 and 11/500,461 and WO 2006/035431 by one of the present inventors, which are incorporated herein by reference and if fully set forth herein, teach a novel class of antimicrobial polymers which are designed based on the knowledge which accumulated over the years on the nature of antimicrobial peptides and the limitations associated with their use, and which are composed of hydrophobic moieties and amino acids. The teachings of these patent applications show that in order to achieve an active antimicrobial agent devoid of the drawbacks of classical antibiotic agents, and those of AMPs, three key attributes of AMPs need to be maintained: a flexible structure, an amphiphatic character and a net positive charge. As successfully presented in the patent applications, these novel active antimicrobial polymers are achieved by the use of positively charged amino acids and the use of non-amino acid hydrophobic moieties, such as, fatty acids and the likes, which will not only achieve the desired amphiphatic trait and resolve the production and maintenance issues limiting the use of polypeptides as drugs, but also alleviate the sever limitations restricting the administration of polypeptides as drugs.

As further demonstrated in U.S. Patent Application Nos. 11/234,183 and 11/500,461 and WO 2006/035431, this newly developed class of polymers has been shown to exhibit high antimicrobial activity, low resistance induction, non-hemolyticity, resistibility to plasma proteases and high affinity to microbial membranes.

### SUMMARY OF THE INVENTION

Although many studies indicate that antimicrobial peptides may be used beneficially as anticancerous agents, most of the presently known antimicrobial peptides and peptidomimetics are of limited utility as therapeutic agents. The need for compounds which have anticancerous activity, and are devoid of the limitations associated presently know chemotherapeutic agents is ever more present, and the concept of providing chemically- and metabolically-stable active peptidomimetic compounds in order to achieve enhanced anticancerous efficacy while preserving the trait of non-MDR induction has been widely recognized.

While the antimicrobial polymers taught in U.S. Patent Application Nos. 11/234,183 and 11/500,461 and WO 2006/035431 indeed exhibit the desired properties and were shown to overcome the limitations associated with common antimicrobial agents as well as AMPs, these polymers were not suggested nor tested as anti-cancerous agents.

The present inventors have now surprisingly found that a class of polymeric compounds, which are based on positively charged amino acid residues and hydrophobic moieties, further exhibit a cytotoxic activity and can thus be utilized as highly efficient anticancerous agents, which are metabolically-stable, non-toxic and non-MDR inducing agents.

Thus, according to one aspect of the present invention there is provided a method of treating cancer in a subject in need thereof, the method if effected by administering to the subject a therapeutically effective amount of a polymer consisting of a plurality of residues, wherein the plurality of residues includes a plurality of amino acid residues and at least one hydrophobic moiety residue, whereas at least one of the hydrophobic moiety residue is being covalently linked to at least two amino acid residues via an amine group of one amino acid residue and via a carboxyl of the other amino acid residue, the polymer being selected from the group consisting of a linear polymer and a cyclic polymer.

According to another aspect of the present invention there is provided a pharmaceutical composition which includes, as an active ingredient, a polymer as described herein, the composition is packaged in a packaging material and identified in print, in or on the packaging material, for use in the treatment of cancer.

According to still another aspect of the present invention there is provided a use of a polymer as described herein in the manufacture of a medicament for the treatment of cancer.

According to some embodiments of the invention described below, the cancer is a multidrug resistant cancer.

According to some embodiments the multidrug resistant cancer is inherent. According to some embodiments the multidrug resistant cancer is acquired.

According to some embodiments the amine group forms a part of the side-chain of at least one of the amino acid residues.

According to some embodiments the amine group is an epsilon amine group of a lysine residue.

According to some embodiments the plurality of residues includes a plurality of amino acid residues, at least one hydrophobic moiety residue, at least one residue that has a first functional group and at least one residue that has a second functional group, whereas the first functional group and the second functional group are covalently linked therebetween, thereby forming the cyclic polymer.

According to some embodiments the first functional group is an amine group and the second functional group is a carboxyl group, or the first functional group is a carboxyl group and the second functional group is an amine group, the first functional group and the second functional group are covalently linked therebetween via a peptide bond.

According to some embodiments the residue that has the amine group as the first or the second functional group is an amino acid residue.

According to some embodiments the amine group is an N-alpha amine group.

According to some embodiments the amine group forms a part of a side chain of the amino acid residue.

According to some embodiments the residue that has the amine group as the first or the second functional group is a hydrophobic moiety residue.

According to some embodiments the residue that has the carboxyl group as the first or the second functional group is an amino acid residue.

According to some embodiments the carboxyl group is a C-alpha carboxyl group.

According to some embodiments the residue that has the carboxyl group as the first or the second functional group is a hydrophobic moiety residue.

According to some embodiments the polymer includes at least two hydrophobic moiety residues, wherein at least one of the hydrophobic moiety residues is linked to the N-alpha of an amino acid residue at the N-terminus thereof and/or the C-alpha of an amino acid residue at the C-terminus thereof.

According to some embodiments the polymer includes at least two hydrophobic moiety residues, wherein at least one of the hydrophobic moiety residues is linked to the side-chain of an amino acid residue.

According to some embodiments the plurality of amino acid residues comprises at least one positively charged amino acid residue.

According to some embodiments at least one hydrophobic moiety residue is linked to at least one of the amino acid residues via a peptide bond.

According to some embodiments at least one hydrophobic moiety residue is linked to each of the amino acid residues via a peptide bond.

According to some embodiments at least one hydrophobic moiety has a carboxylic group at one end thereof and an amine group at the other end thereof.

According to some embodiments the plurality of amino acid residues includes from 2 to 50 amino acid residues.

According to some embodiments the plurality of amino acid residues substantially consists of positively charged amino acid residues.

According some embodiments the positively charged amino acid residues are selected from the group consisting of lysine residues, histidine residues, ornithine residues, arginine residues and combinations thereof. Preferably the positively charged amino acid residues are lysine residues.

According to some embodiments the polymer includes from 1 to 50 hydrophobic moiety residues.

According to some embodiments the hydrophobic moiety residue includes at least one fatty acid residue.

According to some embodiments at least one hydrophobic moiety is a ω-amino-fatty acid residue. Preferably the hydrophobic moiety is selected from the group consisting of 4-amino-butyric acid, 8-amino-caprylic acid and 12-amino-lauric acid.

According some embodiments the polymer further includes at least one additional active agent attached thereto.

According to some embodiments the active agent is selected from the group consisting of a therapeutically active agent, a targeting agent, a labeling agent, a chemosensitization agent and any combination thereof.

According to some embodiments the method of treating further includes administering to the subject at least one additional therapeutically active agent.

According to some embodiments the composition further includes at least one additional therapeutically active agent.

According to some embodiments the medicament is used in combination with at least one additional therapeutically active agent.

According to some embodiments the active agent is an anticancerous agent and/or a chemosensitizing agent.

According to some embodiments the polymer is selected from the group of compounds presented in Table 3 and Table 4 presented hereinbelow.

According to yet another aspect of the present invention there is provided a method of treating cancer in a subject in need thereof, the method is effected by administering to the subject a therapeutically effective amount of a polymer having the general formula I or II:

X-W₀-[A₁-Z₁-D₁]-W₁-[A₂-Z₂-D₂]-W₂-... [An-Zn-Dn]-Wn-Y Formula I

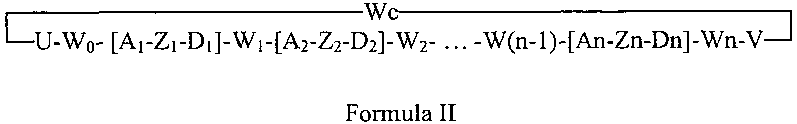

or a pharmaceutically acceptable salt thereof,
n is an integer from 2 to 50;
A₁, A₂, ..., An are each independently an amino acid residue;
D₁, D₂, ..., Dn are each independently a hydrophobic moiety residue or absent, provided that at least one of the D₁, D₂, ..., Dn is the hydrophobic moiety residue;
Z₁, Z₂, ..., Zn and W₀, W₁, W₂, ..., Wn are each independently a linking moiety linking an amino acid residue and a hydrophobic moiety residue, or absent;
X and Y are each independently hydrogen, an amine, an amino acid residue, a hydrophobic moiety residue, has the general Formula I or absent;
W₀ is a linking moiety linking one of the A₁, Z₁ and D₁ to U, or absent;
Wn is a linking moiety linking one of the An, Zn and Dn to V, or absent;
U is selected from the group consisting of a first functional group, an amino acid residue having the first functional group, a hydrophobic moiety residue having the first functional group, and a linking moiety having the first functional group or absent;
V is selected from the group consisting of a second functional group, an amino acid residue having the second functional group, a hydrophobic moiety residue having the second functional group, and a linking moiety having the second functional group or absent; and Wc is a cyclizing moiety.

According to still another aspect of the present invention there is provided a pharmaceutical composition includes, as an active ingredient a polymer having the general formula I or II, the composition is packaged in a packaging material and identified in print, in or on the packaging material, for use in the treatment of cancer.

According to an additional aspect of the present invention there is provided a use of a polymer having the general formula I or II in the manufacture of a medicament for treating cancer.

According to some embodiments of the invention described below, X is a hydrophobic moiety residue.

According to some embodiments Y is a hydrophobic moiety residue.

According to some embodiments each of X and Y is a hydrophobic moiety residue.

According to some embodiments at least one of the W₀, W₁, W₂, ... Wₙ and the Z₁, Z₂, ...Zₙ is a peptide bond.

According to some embodiments Wc is a peptide bond.

According to some embodiments each of the W₀, W₁, W₂, ...Wₙ and Z₁, Z₂, ...Zₙ is a peptide bond.

According to some embodiments at least one of the D₁, D₂, ..., Dn is a ω-amino-fatty acid residue.

According to some embodiments at least one of the hydrophobic moiety comprises at least one hydrocarbon chain.

According to some embodiments each of the D₁, D₂, ..., Dn is a 8-amino-caprylic acid.

According to some embodiments n is an integer from 5 to 7.

According to some embodiments X is a dodecanoic acid residue and Y is an amine.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the patent specification, including definitions, will control. In addition, the materials, methods, and examples are illustrative only and not intended to be limiting.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:
FIGs. 1a-f present an illustration of a putative model for the mechanism of action of antimicrobial and anticancerous peptides and peptidomimetic polymers (jointly referred to as AMPs), showing positively charged AMPs, illustrated as green and red twiddled lines decorated with "plus" signs, bind to the outer leaflet of the bilayer lipid membrane (BLM) surface, and interact with the negatively charged phospholipids head groups (Figure 1a); thinning-off and expanding of the outer leaflet which leads to osmotic pressure strain on the weakened bilayer (Figure1b); AMP insertion through the fractured outer leaflet of the membrane into the core of the bilayer and formation of transient pores (Figure 1c); translocation of AMPs to the inner leaflet (Figure 1d); attainment of access and targeting of essential intracellular structures and functions (Figure 1e); and/or membrane permeation and destruction of the cell's structure (Figure 1f);

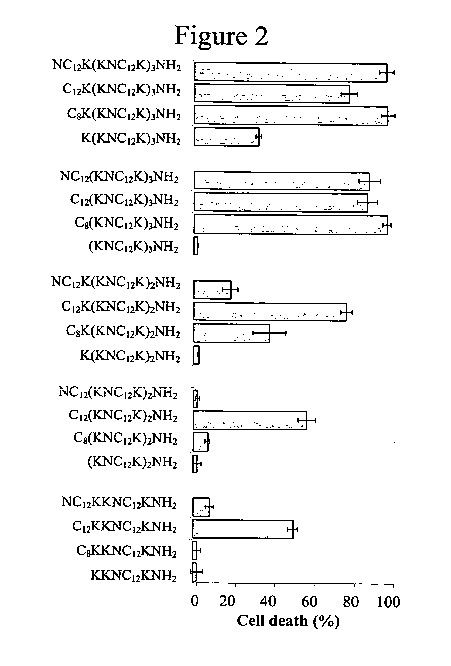

FIG. 2 presents a comparative bar graph showing the percent cell death as a result of exposure of MCF-7 cells to 5 series of exemplary polymers according to the present embodiments, each series being of 4 polymers, differing in length and various chemical groups, each at a concentration of 16 µM, demonstrating the anticancerous potential of the polymers and displaying the structure-activity relations and particularly the contribution of the length and net positive charge of the polymer on the cancerous-cell killing capacity thereof;

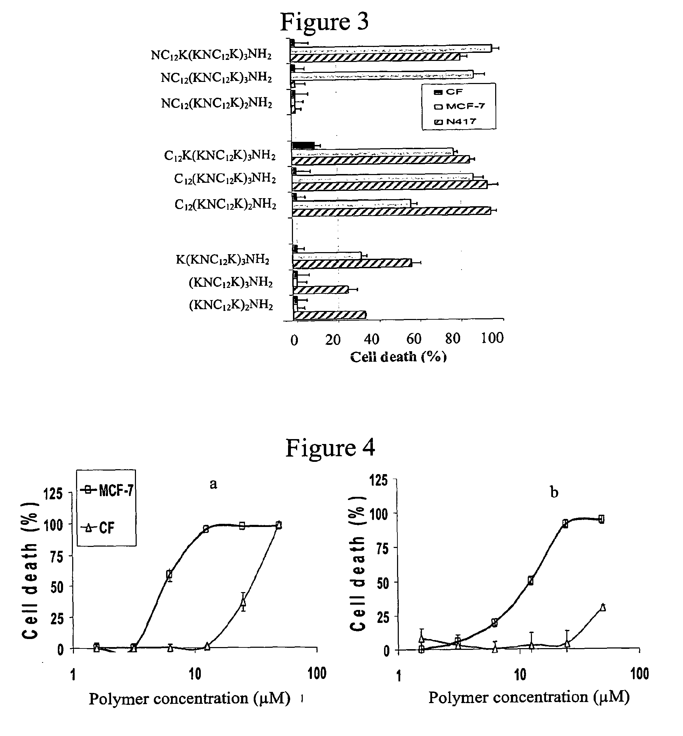

FIG. 3 presents a comparative bar graph showing the percent cell death as a result of exposure of rat cardiac fibroblast (marked by solid dark gray bars), human MCF-7 cells (marked by solid light gray bars) and human N-417 cells (marked by striped bars) to 3 series of exemplary polymers according to the present embodiments, each series being of 3 polymers, differing in length and various terminal groups, each at a concentration of 16 µM, demonstrating the selective in vitro activity of the polymers against non-cancerous cells and two types of cancers;
FIGs. 4a-b present comparative plots demonstrating the dose-dependent activity of two exemplary anticancerous polymers, C₈K(KNC₁₂K)₃NH₂ (Figure 4a) and C₁₂(KNC₁₂K)₂NH₂ (Figure 4b), according to the present embodiments, showing LC₅₀ values of 5.5 µM and 11 µM against MCF-7 cells (marked by rectangles), and 25 µM and 67 µM against non-cancerous CF cells (marked by triangles), respectively, demonstrating the selective activity exhibited by the polymers;

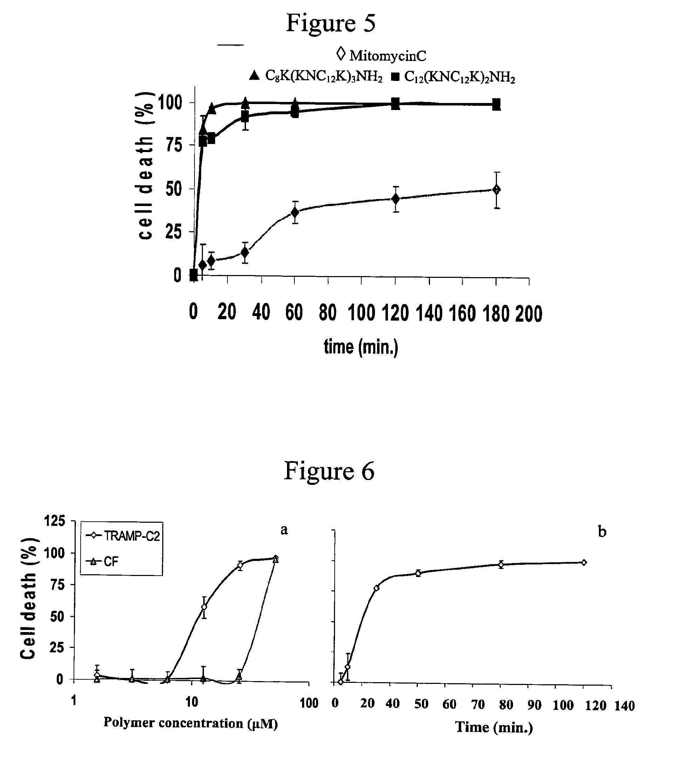

FIG. 5 presents comparative plots demonstrating the kinetic cytotoxic activity of C₈K(KNC₁₂K)₃NH₂ (marked by triangles) and C₁₂(KNC₁₂K)₂NH₂ (marked by rectangles), two exemplary anticancerous polymers according to the present embodiments, on MCF-7 cells, as compared with kinetic cytotoxic activity of mitomycin C (marked by diamonds), as determined for each at a concentration of 8 times their LC₅₀ value;
FIG. 6 presents the cytotoxic aptitude of C₁₂(KNC₁₂K)₃NH₂, an exemplary anticancerous polymer according to the present embodiments, showing the polymer's selectivity towards killing cancerous cells, exhibiting an LC₅₀ value of 11 µM towards TRAMP-C2 cells (Figure 6a, marked by diamonds), as compared to a much higher LC₅₀ value of 38 µM towards benign CF cells (Figure 6a, marked by triangles) measured in a dose-dependent comparative plot, and presenting the rapid rate of cytotoxicity in a time-dependent activity plot (Figure 6b);

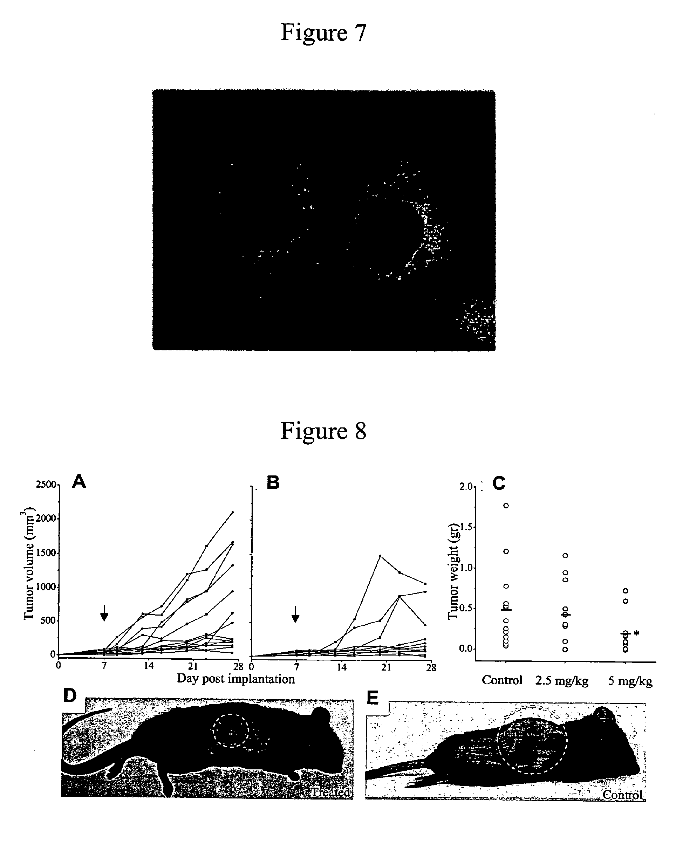

FIG. 7 presents a photograph taken on a confocal fluorescence microscope (magnification: 1 cm = 5 microns) of two cells in a TRAMP-C2 cell culture treated for 30 minutes with 20 µM of NC₁₂(KNC₁₂K)₃NH₂, an exemplary polymer according to the present embodiments, labeled with 7-fluoro-4-nitrobenzo-2-oxo-1,3-diazole (NBD-F), and further incubated with a mitochondrial specific marker MitoTracker Red, showing the cellular localization the polymer inside the cell and distribution throughout the cytoplasm;
FIGs. 8A-E illustrate the effect of NC₁₂K(KNC₁₂K)₃, an exemplary polymer according to the present embodiments, in the treatment of TRAMP-C2 allograft tumors induced by injection in C57BL/6 mice, showing tumor growth curves for each mouse treated by a daily injection of a blank control PBS (n=14) (Figure 8A) or 5 mg/kg of NC₁₂K(KNC₁₂K)₃ (n=11) (Figure 8B) wherein treatment commenced when tumor volume reached 40-50 mm³ (indicated by an arrow in both Figures 8A and B), showing a weight distribution graph of the measured tumors (Figure 8C) extracted at the 28^{th} day post tumor inoculation from mice treated with PBS (control) or NC₁₂K(KNC₁₂K)₃, at the specified doses (2.5 mg/kg and 5 mg/kg), and showing representative tumor bearing mice which were treated with NC₁₂K(KNC₁₂K)₃ (Figure 8D), or treated with the PBS control (Figure 8E);

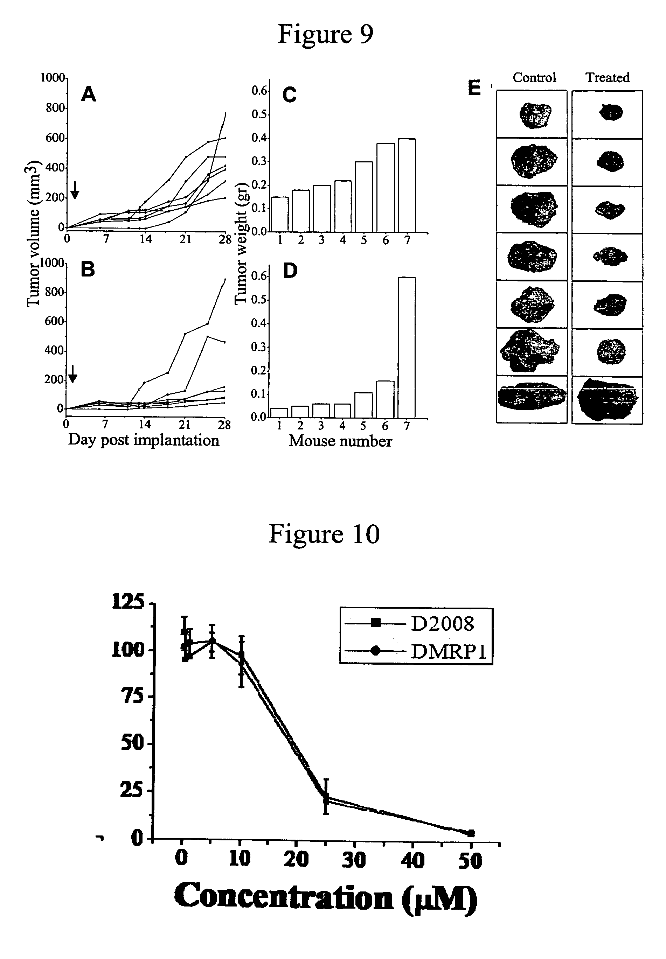

FIGs. 9A-E illustrate the effect of early treatment of C57BL/6 mice bearing TRAMP-C2 allograft tumors with NC₁₂K(KNC₁₂K)₃, which started 24 hours after inoculation, (indicated by an arrow in Figures 9A and B), showing growth curves for each mouse when treated by a daily injection with PBS (n=7) (Figure 9A) or 5 mg/kg of NC₁₂K(KNC₁₂K)₃ (n=7) (Figure 9B), showing two weight distribution graphs of tumors extracted from mice treated with PBS (Figure 9C) or NC₁₂K(KNC₁₂K)₃ (Figure 9D) on the 28^{th} day post inoculation, and showing a series of photographs of the extracted and measured tumors (Figure 9E);
FIG. 10 presents a comparative plot of the dose dependent cytotoxic effect of NC₁₂K(KNC₁₂K)₃ against ovarian carcinoma 2008 drug-sensitive wild type cells (shown in black rectangles) and against drug-resistant 2008/MRP1 cells (in red circles) after overnight incubations, presenting the cells viability as percentages of the control as a function of doses, wherein each data point represents the mean of two experiments performed in duplicate cultures for each drug concentration, and the vertical bars represent one SD, showing that NC₁₂K(KNC₁₂K)₃ displayed similar potency against the drug-sensitive cells as compared to its potency against the drug-resistant cells (LC₅₀= about 15 µM); and

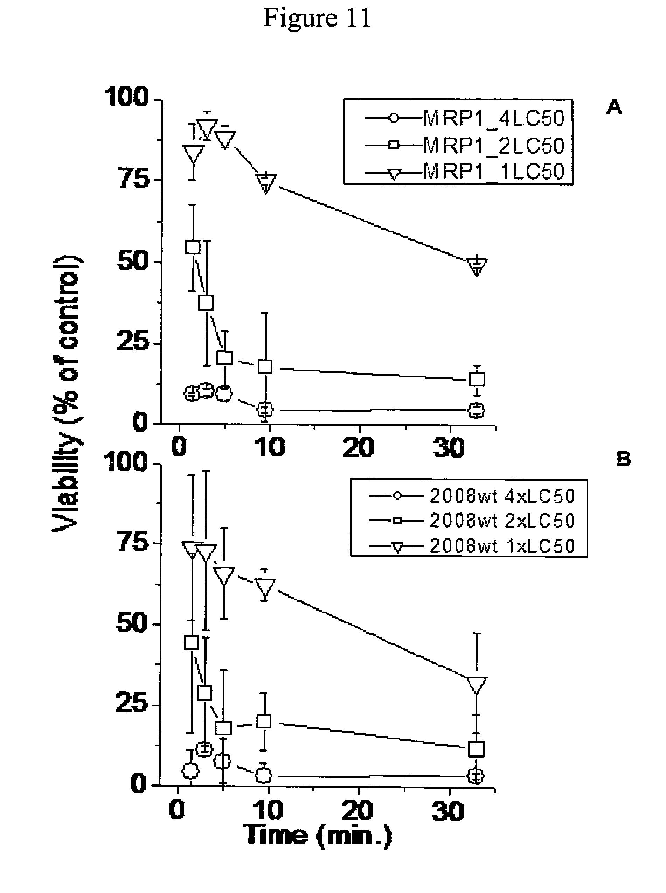

FIGs. 11A-B present two comparative plots of the time dependent cytotoxic effect (time-kill curves) of NC₁₂K(KNC₁₂K)₃ against the drug-resistant 2008/MRP1 cells (Figure 11 A) and the drug-sensitive human ovarian carcinoma 2008 cells (Figure 11B), wherein each data point represents the mean of two experiments performed in duplicate cultures for each drug concentration, and the vertical bars represent one SD, showing that the cytotoxic effect is extremely rapid in terms of minutes.

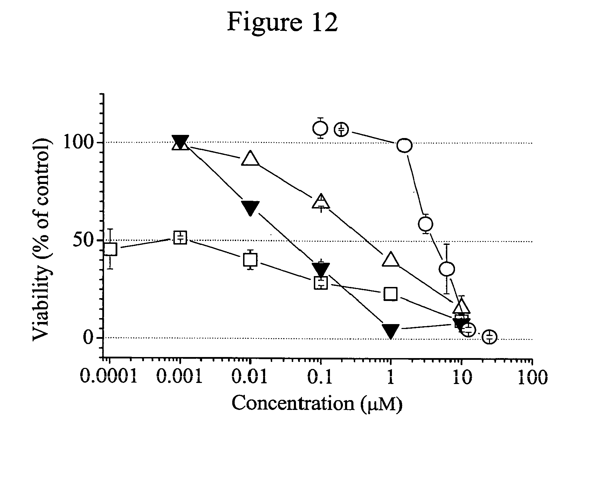

FIG. 12 presents a comparative plot of the dose dependent cytotoxic effect of doxorubicin against drug-sensitive ovarian carcinoma 2008 cells (black triangles), doxorubicin against drug-resistant 2008/MRP1 cells (white triangles), NC₁₂K(KNC₁₂K)₃, an exemplary polymer according to the present embodiments, against drug-resistant 2008/MRP1 cells (white triangles), and the cytotoxic effect of the combination of doxorubicin (varying dose) and NC₁₂K(KNC₁₂K)₃ (constant 4 µM concentration) against drug-resistant 2008/MRP1 cells (white rectangles), all after 3-day cultures (each data point represents the mean of two experiments performed in duplicate cultures for each drug concentration, and the vertical bars represent one SD).

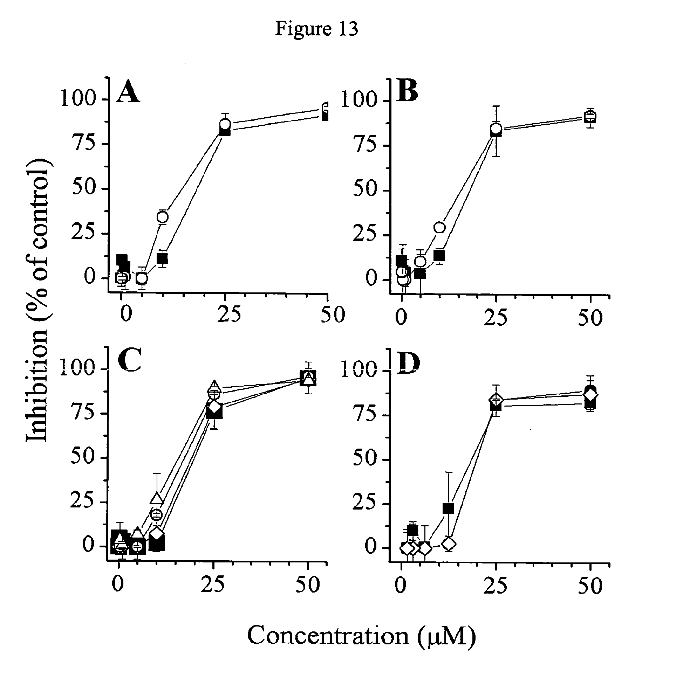

FIGs. 13A-D present comparative plots, showing the dose dependent cytotoxic effect of NC₁₂K(KNC₁₂K)₃NH₂, an exemplary polymer according to the present embodiments, as measured using the XTT assay on resistant and sensitive cells, namely against AA8 and EMTR1 cell-lines (marked in black rectangles and white circles respectively in Figure 13A); A549 and K1.5 cell-lines (marked in black rectangles and white circles respectively in Figure 13B); 2008, MRP1, MRP2 and MRP3 cell-lines (marked in black rectangles, white diamonds, white circles and white triangles respectively in Figure 13C); and against HEK, MRP4 and MRP5 cell-lines (marked in black rectangles, black circles and white diamonds in Figure 13D).

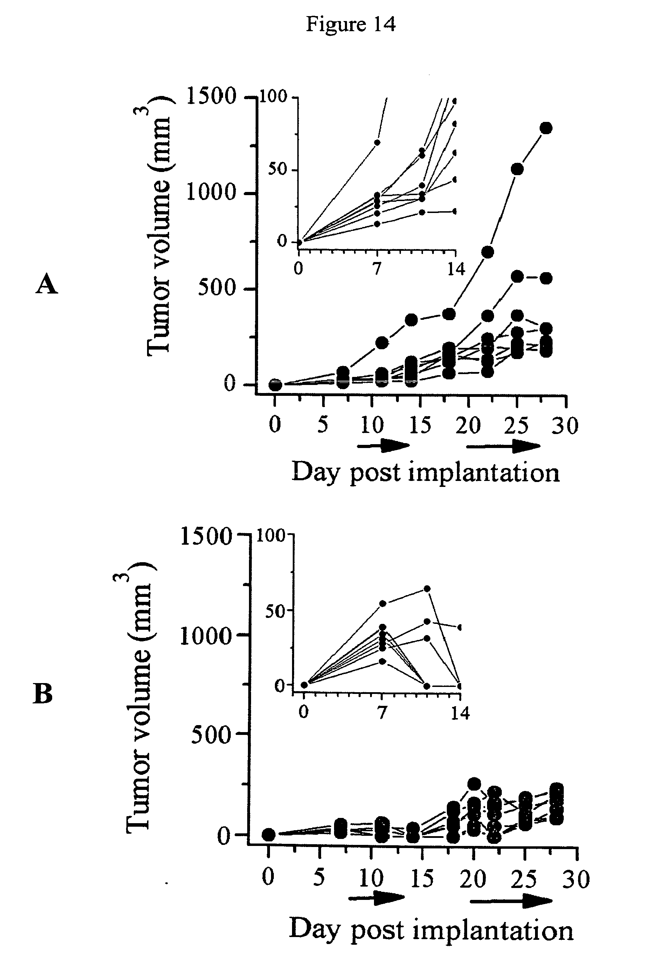

FIGs. 14A-B present comparative plots showing the tumor growth curves as measured for each mouse when treated intratumorally with control PBS (Figure 14A) or 5 mg/kg of NC₁₂K(KNC₁₂K)₃NH₂, an exemplary polymer according to the present embodiments, (Figure 14B), and showing the tumor growth in the first 14 days after cells implantation of the PBS treated mice (insert in Figure 14A) and the polymer treated mice (insert in Figure 14B).

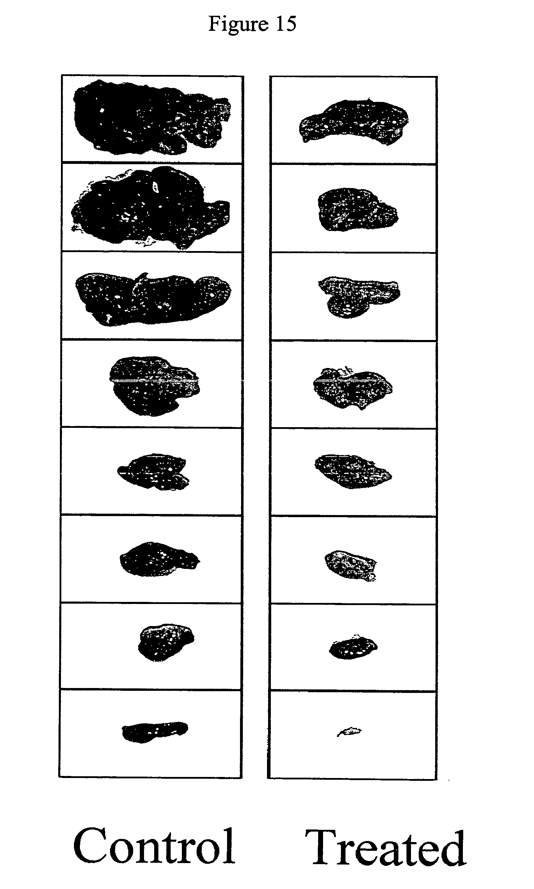

FIG. 15 presents two series of color photographs, showing tumors which were resected from syngenic C57BL/6J mice which were grafted with tumorigenic TRAMP-C2 murine prostate cells and then treated with PBS as control (left-side series) and NC₁₂K(KNC₁₂K)₃NH₂, and exemplary anticancerous polymer as presented herein, and showing the notable anticancerous effect of the polymer *in vivo.*

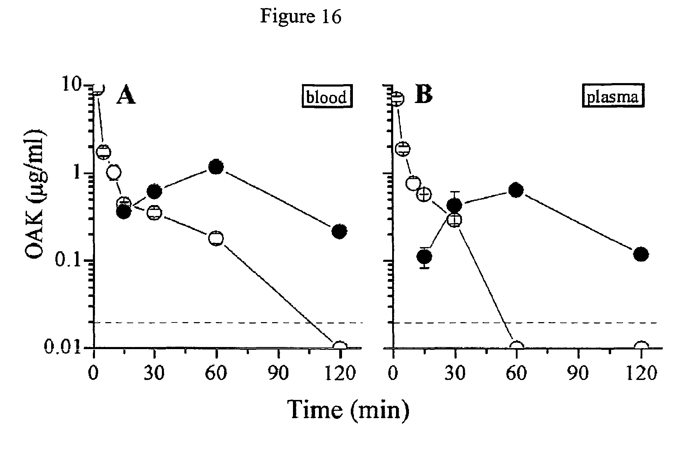

FIG. 16 presents comparative plots, showing the concentrations of NC₁₂K(KNC₁₂K)₃NH₂, an exemplary polymer according to the present embodiments, as determined after 100 µg/mouse intraperitoneal administration (marked in black circles in Figure 16) and intravenous administration (marked in white circles in Figure 16) of the polymer in whole blood (Figure 16A) and in plasma (Figure 16B) after 30 minutes incubation at 25 °C in the extraction buffer (dashed line defines the limit of detection).

### DESCRIPTION OF EMBODIMENTS OF THE INVENTION

The present invention, in some embodiments thereof, relates to the use of antimicrobial polymeric agents in the treatment of cancer. Specifically, the present invention, in some embodiments thereof, relates to methods and uses of polymeric compounds, composed of a plurality of amino acid residues and one or more hydrophobic moieties linking therebetween, in the treatment of cancer.

The principles and operation of the present invention may be better understood with reference to the figures and accompanying descriptions.

Before explaining at least one embodiment of the invention in detail, it is to be understood that the invention is not limited in its application to the details set forth in the following description or exemplified by the Examples. The invention is capable of other embodiments or of being practiced or carried out in various ways. Also, it is to be understood that the phraseology and terminology employed herein is for the purpose of description and should not be regarded as limiting.

As discussed above, the use of the currently practiced cancer therapies is severely limited, mainly by the adverse side effects associated therewith that often result from the non-selectivity of the agent used and/or by the development of multidrug resistance (MDR).

As further discussed above, albeit the fact that several peptides and AMPs were found to possess anticancerous activities, these classes of compounds suffer from the limitations associated with peptide production, maintenance and modes of clinical administration for therapeutic use, described hereinabove.

U.S. Patent Application Nos. 11/234,183 and 11/500,461 and WO 2006/035431, by one of the present inventors, which are incorporated herein by reference as if fully set forth herein, teach a novel class of antimicrobial polymeric agents which were designed to exert antimicrobial activity while being chemically and pharmaceutically stable, non-toxic and non-resistance inducing, as well as methods of preparing of these agents, pharmaceutical compositions containing same and a method of treating medical conditions associated with pathological microorganisms. These antimicrobial polymeric agents were shown to be non-hemolyticity and to exhibit resistibility to plasma proteases.

The design paradigms of these antimicrobial polymeric agents were based on the knowledge which accumulated over the years on the nature of antimicrobial peptides and the limitations associated with their use, and included three key attributes, namely a flexible structure, an amphiphatic character and a net positive charge.

Thus, polymeric agents composed of a plurality of amino acid residues and one or more hydrophobic moieties, each linking two amino acid residues and/or being attached to a terminus residue, have been designed and successfully practiced as antimicrobial agents.

The present inventors have now surprisingly uncovered that such polymeric agents exhibit anticancerous activity and are advantageously further characterized as being stable, having high selectivity towards cancerous cells, and as exhibiting low levels of MDR induction, and hence can serve as a potent cancer therapy which obviate the limitations associated with current methodologies.

Thus, according to one aspect of the present invention there is provided a method of treating cancer in a subject in need thereof. The method, according to this aspect of the present invention is effected by administering to the subject a therapeutically effective amount of one or more of the anticancerous polymeric agents presented herein, henceforth the polymers.

Each of the polymers according to the present embodiments, comprises two or more monomers, also referred to herein interchangeably as residues, which include two or more amino acid residues and one or more hydrophobic moiety residues, as these terms are defined hereinbelow, whereas at least one of the hydrophobic moiety residues is being covalently linked to at least two amino acid residues via an amine group of one amino acid and via a carboxyl group of another amino acid residue. The polymer can be a linear polymer or a cyclic polymer, as these terms are defined hereinbelow. Therefore, the polymers described herein each is comprised of a linear or cyclic chain made of a sequence of amino acid residues, interrupted by one or more hydrophobic moiety residues

The present embodiments further encompass any enantiomers, prodrugs, solvates, hydrates and/or pharmaceutically acceptable salts of the polymers described herein.

As used herein, the terms "treating" and "treatment" include abrogating, substantially inhibiting, slowing or reversing the progression of a condition, substantially ameliorating clinical or aesthetical symptoms of a condition or substantially preventing the appearance of clinical or aesthetical symptoms of a condition.

As used herein, the phrase "therapeutically effective amount" describes an amount of the polymer being administered which will relieve to some extent one or more of the symptoms of the condition being treated.

As used herein, the term "enantiomer" refers to a stereoisomer of a polymer that is superposable with respect to its counterpart only by a complete inversion/reflection (mirror image) of each other. Enantiomers are said to have "handedness" since they refer to each other like the right and left hand. Enantiomers have identical chemical and physical properties except when present in an environment which by itself has handedness, such as all living systems.

The term "prodrug" refers to an agent, which is converted into the active polymer (the active parent drug) *in vivo.* Prodrugs are typically useful for facilitating the administration of the parent drug. They may, for instance, be bioavailable by oral administration whereas the parent drug is not. A prodrug may also have improved solubility as compared with the parent drug in pharmaceutical compositions. Prodrugs are also often used to achieve a sustained release of the active compound *in vivo.* An example, without limitation, of a prodrug would be a compound of the present invention, having one or more carboxylic acid moieties, which is administered as an ester (the "prodrug"). Such a prodrug is hydrolyzed *in vivo,* to thereby provide the free compound (the parent drug). The selected ester may affect both the solubility characteristics and the hydrolysis rate of the prodrug.

The term "solvate" refers to a complex of variable stoichiometry (e.g., di-, tri-, tetra-, penta-, hexa-, and so on), which is formed by a solute (the polymer of the present embodiments) and a solvent, whereby the solvent does not interfere with the biological activity of the solute. Suitable solvents include, for example, ethanol, acetic acid and the like.

The term "hydrate" refers to a solvate, as defined hereinabove, where the solvent is water.

The phrase "pharmaceutically acceptable salt" refers to a charged species of the parent polymer and its counter ion, which is typically used to modify the solubility characteristics of the parent compound and/or to reduce any significant irritation to an organism by the parent polymer, while not abrogating the biological activity and properties of the administered polymer. An example, without limitation, of a pharmaceutically acceptable salt would be a carboxylate anion and a cation such as, but not limited to, ammonium, sodium, potassium and the like.

As used herein throughout the term "amino acid" or "amino acids" is understood to include the 20 genetically coded amino acids; those amino acids often modified post-translationally *in vivo,* including, for example, hydroxyproline, phosphoserine and phosphothreonine; and other unusual amino acids including, but not limited to, 2-aminoadipic acid, hydroxylysine, isodesmosine, nor-valine, norleucine and ornithine. Furthermore, the term "amino acid" includes both D- and L-amino acids and other non-naturally occurring amino acids.

Tables 1 and 2 below list the genetically encoded amino acids (Table 1) and non-limiting examples of non-conventional/modified amino acids (Table 2) which can be used with the present invention.

**Table 1**

| **Amino acid** | **Three-Letter Abbreviation** | **One-letter Symbol** |
|---|---|---|
| Alanine | Ala | A |
| Arginine | Arg | R |
| Asparagine | Asn | N |
| Aspartic acid | Asp | D |
| Cysteine | Cys | C |
| Glutamine | Gln | Q |
| Glutamic acid | Glu | E |
| Glycine | Gly | G |
| Histidine | His | H |
| Isoleucine | Iie | I |
| Leucine | Leu | L |
| Lysine | Lys | K |
| Methionine | Met | M |
| Phenylalanin | Phe | F |
| e | | |
| Proline | Pro | P |
| Serine | Ser | S |
| Threonine | Thr | T |
| Tryptophan | Trp | W |
| Tyrosine | Tyr | Y |
| Valine | Val | V |

**Table 2**

| **Non-conventional amino acid** | **Code** | **Non-conventional amino acid** | **Code** |
|---|---|---|---|
| α-aminobutyric acid | Abu | L-N-methylalanine | Nmala |
| α-amino-α-methylbutyrate | Mgabu | L-N-methylarginine | Nmarg |
| aminocyclopropane-carboxylate | Cpro | L-N-methylasparagine | Nmasn |
| aminoisobutyric acid | Aib | L-N-methylaspartic acid | Nmasp |
| aminonorbornyl-carboxylate | Norb | L-N-methylcysteine | Nmcys |
| Cyclohexylalanine | Chexa | L-N-methylglutamine | Nmgin |
| Cyclopentylalanine | Cpen | L-N-methylglutamic acid | Nmglu |
| D-alanine | Dal | L-N-methylhistidine | Nmhis |
| D-arginine | Darg | L-N-methylisolleucine | Nmile |
| D-aspartic acid | Dasp | L-N-methylleucine | Nmleu |
| D-cysteine | Dcys | L-N-methyllysine | Nmlys |
| D-glutamine | Dgln | L-N-methylmethionine | Nmmet |
| D-glutamic acid | Dglu | L-N-methylnorieucine | Nmnle |
| D-histidine | Dhis | L-N-methylnorvaline | Nmnva |
| D-isoleucine | Dile | L-N-methylornithine | Nmorn |
| D-leucine | Dleu | L-N-methylphenylalanine | Nmphe |
| D-lysine | Dlys | L-N-methylproline | Nmpro |
| D-methionine | Dmet | L-N-methylserine | Nmser |
| D/L-ornithine | D/Lorn | L-N-methylthreonine | Nmthr |
| D-phenylalanine | Dphe | L-N-methyltryptophan | Nmtrp |
| D-proline | Dpro | L-N-methyltyrosine | Nmtyr |
| D-serine | Dser | L-N-methylvaline | Nmval |
| D-threonine | Dthr | L-N-methylethylglycine | Nmetg |
| D-tryptophan | Dtrp | L-N-methyl-t-butylglycine | Nmtbug |
| D-tyrosine | Dtyr | L-norleucine | Nle |
| D-valine | Dval | L-norvaline | Nva |
| D-α-methylalanine | Dmala | α-methyl-aminoisobutyrate | Maib |
| D-α-methylarginine | Dmarg | α-methyl-γ-aminobutyrate | Mgabu |
| D-α-methylasparagine | Dmasn | α-methylcyclohexylalanine | Mchexa |
| D-α-methylaspartate | Dmasp | α-methylcyclopentylalanine | Mcpen |
| D-α-methylcysteine | Dmcys | α-methyl-α-napthylalanine | Manap |
| D-α-methylglutamine | Dmgln | α-methylpenicillamine | Mpen |
| D-α-methylhistidine | Dmhis | N-(4-aminobutyl)glycine | Nglu |
| D-α-methylisoleucine | Dmile | N-(2-aminoethyl)glycine | Naeg |
| D-α-methylleucine | Dmleu | N-(3-aminopropyl)glycine | Norn |
| D-α-methyllysine | Dmlys | N-amino-α-methylbutyrate | Nmaabu |
| D-α-methylmethionine | Dmmet | α-napthylalanine | Anap |
| D-a-methylomithine | Dmom | N-benzylglycine | Nphe |
| D-α-methylphenylalanine | Dmphe | N-(2-carbamylethyl)glycine | Ngln |
| D-α-methylproline | Dmpro | N-(carbamylmethyl)glycine | Nasn |
| D-α-methylserine | Dmser | N-(2-carboxyethyl)glycine | Nglu |
| D-α-methylthreonine | Dmthr | N-(carboxymethyl)glycine | Nasp |
| D-α-methyltryptophan | Dmtrp | N-cyclobutylglycine | Ncbut |
| D-α-methyltyrosine | Dmty | N-cycloheptylglycine | Nchep |
| D-α-methylvaline | Dmval | N-cyclohexylglycine | Nchex |
| D-α-methylalnine | Dnmala | N-cyclodecylglycine | Ncdec |
| D-α-methylarginine | Dnmarg | N-cyclododeclglycine | Ncdod |
| D-α-methylasparagine | Dnmasn | N-cyclooctylglycine | Ncoct |
| D-α-methylasparatate | Dnmasp | N-cyclopropylglycine | Ncpro |
| D-α-methylcysteine | Dnmcys | N-cycloundecylglycine | Ncund |
| D-N-methylleucine | Dnmleu | N-(2,2-diphenylethyl)glycine | Nbhm |
| D-N-methyllysine | Dnmlys | N-(3,3-diphenylpropyl)glycine | Nbhe |
| N-methylcyclohexylalanine | Nmchexa | N-(3-indolylyethyl) glycine | Nhtrp |
| D-N-methylornithine | Dnmorn | N-methyl-γ-aminobutyrate | Nmgabu |
| N-methylglycine | Nala | D-N-methylmethionine | Dnmmet |
| N-methylaminoisobutyrate | Nmaib | N-methylcyclopentylalanine | Nmcpen |
| N-(1-methylpropyl)glycine | Nile | D-N-methylphenylalanine | Dnmphe |
| N-(2-methylpropyl)glycine | Nile | D-N-methylproline | Dnmpro |
| N-(2-methylpropyl)glycine | Nleu | D-N-methylserine | Dnmser |
| D-N-methyltryptophan | Dnmtrp | D-N-methylserine | Dnmser |
| D-N-methyltyrosine | Dnmtyr | D-N-methylthreonine | Dnmthr |
| D-N-methylvaline | Dnmval | N-(1-methylethyl)glycine | Nva |
| γ-aminobutyric acid | Gabu | N-methyla-napthylalanine | Nmanap |
| L-t-butylglycine | Tbug | N-methylpenicillamine | Nmpen |
| L-ethylglycine | Etg | N-(*p*-hydroxyphenyl)glycine | Nhtyr |
| L-homophenylalanine | Hphe | N-(thiomethyl)glycine | Ncys |
| L-α-methylarginine | Marg | penicillamine | Pen |
| L-α-methylaspartate | Masp | L-α-methylalanine | Mala |
| L-α-methylcysteine | Mcys | L-α-methylasparagine | Masn |
| L-α-methylglutamine | Mgln | L-α-methyl-t-butylglycine | Mtbug |
| L-α-methylhistidine | Mhis | L-methylethylglycine | Metg |
| L-α-methylisoleucine | Mile | L-α-methylglutamate | Mglu |
| D-N-methylglutamine | Dnmgln | L-α-methylhomo phenylalanine | Mhphe |
| D-N-methylglutamate | Dnmglu | N-(2-methylthioethyl)glycine | Nmet |
| D-N-methylhistidine | Dnmhis | N-(3-guanidinopropyl)glycine | Narg |
| D-N-methylisoleucine | Dnmile | N-(1-hydroxyethyl)glycine | Nthr |
| D-N-methylleucine | Dnmleu | N-(hydroxyethyl)glycine | Nser |
| D-N-methyllysine | Dnmlys | N-(imidazolylethyl)glycine | Nhis |
| N-methylcyclohexylalanine | Nmchexa | N-(3-indolylyethyl)glycine | Nhtrp |
| D-N-methylornithine | Dnmorn | N-methyl-γ-aminobutyrate | Nmgabu |
| N-methylglycine | Nala | D-N-methylmethionine | Dnmmet |
| N-methylaminoisobutyrate | Nmaib | N-methylcyclopentylalanine | Nmcpen |
| N-(1-methylpropyl)glycine | Nile | D-N-methylphenylalanine | Dnmphe |
| N-(2-methylpropyl)glycine | Nleu | D-N-methylproline | Dnmpro |
| D-N-methyltryptophan | Dnmtrp | D-N-methylserine | Dnmser |
| D-N-methyltyrosine | Dnmtyr | D-N-methylthreonine | Dnmthr |
| D-N-methylvaline | Dnmval | N-(1-methylethyl)glycine | Nval |
| γ-aminobutyric acid | Gabu | N-methyla-napthylalanine | Nmanap |
| L-t-butylglycine | Tbug | N-methylpenicillamine | Nmpen |
| L-ethylglycine | Etg | N-(p-hydroxyphenyl)glycine | Nhtyr |
| L-homophenylalanine | Hphe | N-(thiomethyl)glycine | Ncys |
| L-α-methylarginine | Marg | penicillamine | Pen |
| L-α-methylaspartate | Masp | L-α-methylalanine | Mala |
| L-α-methylcysteine | Mcys | L-α-methylasparagine | Masn |
| L-α-methylglutamine | Mgln | L-α-methyl-t-butylglycine | Mtbug |
| L-α-methylhistidine | Mhis | L-methylethylglycine | Metg |
| L-α-methylisoleucine | Mile | L-α-methylglutamate | Mglu |
| L-α-methylleucine | Mleu | L-α-methylhomophenylalanine | Mhphe |
| L-α-methylmethionine | Mmet | N-(2-methylthioethyl)glycine | Nmet |
| L-α-methylnorvaline | Mnva | L-α-methyllysine | Mlys |
| L-α-methylphenylalanine | Mphe | L-α-methylnorleucine | Mnle |
| L-α-methylserine | mser | L-α-methylornithine | Morn |
| L-α-methylvaline | Mtrp | L-α-methylproline | Mpro |
| L-α-methylleucine | Mval Nnbhm | L-α-methylthreonine | Mthr |
| N-(N-(2,2-diphenylethyl)carbamylmethyl-glycine | Nnbhm | L-α-methyltyrosine | Mtyr |
| 1-carboxy-1-(2,2-diphenyl ethylamino)cyclopropane | Nmbc | L-N-methylhomophenylalanine | Nmhphe |
| N-(N-(3,3-diphenylpropyl)carbamylmethyl(1)g lycine | Nnbhe | D/L-citrulline | D/Lctr |

As is well accepted in the art in the molecular context, the term "residue", as used herein, refers to a portion, and typically a major portion of a molecular entity, such as molecule or a part of a molecule such as a group, which has underwent a chemical reaction and is now covalently linked to another molecular entity. In the context of the present invention, a residue is an equivalent term to a monomer comprising the polymer. For example, the molecular entity can be an amino acid molecule, and the portion of the amino acid which forms a part of a polypeptide chain (a polymer) after the formation of the polypeptide chain, is an amino acid residue (a monomer). An amino acid residue is therefore that part of an amino acid which is present in a peptide sequence upon reaction of, for example, an alpha-amine group thereof with a carboxylic group of an adjacent amino acid in the peptide sequence, to form a peptide amide bond and/or of an alpha-carboxylic acid group thereof with an alpha-amine group of an adjacent amino acid in the peptide sequence, to form a peptide amide bond. Similarly, the term "residue" refers to the major part of a hydrophobic moiety, such as, for example the acyl part of a fatty acid.

As used herein, the phrase "moiety" describes a part, and preferably a major part of a chemical entity or compound, which typically has certain functionality or distinguishing features.

As used herein, the phrase "hydrophobic moiety" describes a chemical moiety that has a minor or no affinity to water, that is, which has a low or no dissolvability in water and often in other polar solvents. Exemplary suitable hydrophobic moieties for use in the context of the present embodiments, include, without limitation, hydrophobic moieties that consist predominantly of one or more hydrocarbon chains and/or aromatic rings, and one or more functional groups which may be non-hydrophobic, but do not alter the overall hydrophobicity of the hydrophobic moiety. Representative examples include, without limitation, fatty acids, hydrophobic amino acids (amino acids with hydrophobic side-chains), alkanes, alkenes, aryls and the likes, as these terms are defined herein, and any combination thereof.

The term "side-chain", as used herein with reference to amino acids, refers to a chemical group which is attached to the α-carbon atom of an amino acid. The side-chain is unique for each type of amino acid and typically does not take part in forming the peptide bond in a naturally occurring protein or polypeptide, but can be used to form a link between monomers in the polymer presented herein in cases the side-chain comprises a suitable functional group. For example, the side chain for glycine is hydrogen, for alanine it is methyl, for valine it is isopropyl, for phenylalanine it is benzyl, and the side chain for lysine can be regarded as an amino-butyl group, e.g., having an available amine group. For the specific side chains of all amino acids reference is made to A. L. Lehninger's text on Biochemistry (see, chapter 4).

The term "linear" as used herein in the context of the polymers, refers to a non-cyclic polymer, i.e., a polymer which have two termini and its backbone or amino-acid side-chains do not form a closed ring.

According to certain embodiments of the present invention, the linear or cyclic polymer comprises a plurality of amino acid residues and one or more hydrophobic moiety residues as described hereinabove, wherein at least one of the hydrophobic moiety residues is being covalently linked to one of the amino acid residues via an amine group in the side-chain thereof. According to preferred embodiments, the amine group in the side-chain of the amino acid residue is the epsilon amine group of a lysine residue.

The term "cyclic" as used herein in the context of the polymer, refers to a polymer that comprises an intramolecular covalent bond between two non-adjacent residues (monomers) therein, forming a cyclic polymer ring.

In the context of the present embodiments the polymer comprises residues of amino acids and hydrophobic moieties which constitute the monomers of the polymer. The term residue is meant to encompass other chemical moieties which form a part of the polymer, and which do not fall under the definition of amino acid or hydrophobic moiety, as these are defined herein. For example, the cyclic polymer may be "closed" or cyclized by means of a multifunctional or bifunctional moiety that will form a part of the cyclic polymer once it is cyclized.

According to some embodiments with respect to the cyclic polymer, the polymer includes at least one residue that has a functional group, which is referred to herein as the first functional group, and at least one residue that has a second functional group, whereas the first and second functional groups are covalently linked therebetween, thereby forming a cyclic polymer.

As used herein, the phrase "functional group" describes a chemical group that is capable of undergoing a chemical reaction that typically leads to a bond formation. The bond, according to the present embodiments, is preferably a covalent bond. Chemical reactions that lead to a bond formation include, for example, nucleophilic and electrophilic substitutions, nucleophilic and electrophilic addition reactions, addition-elimination reactions, cycloaddition reactions, rearrangement reactions and any other known organic reactions that involve a functional group.

The first and second functional groups may form a part of an amino acid residue and/or a hydrophobic moiety residue in the polymer, or any other element in the polymer which does not fall under the definition of amino acid or hydrophobic moiety, such as, for example, a linking moiety. The first and second functional groups are selected such that they are capable of forming a covalent bond therebetween or therefrom. For example, either the first or the second functional group can be a binding pair of an amine and a carboxyl which form an amide (peptide bond), a hydroxyl and a carboxyl which form an ester, or a an amine and an aldehyde which form an imine (Schiff base).

According to some embodiments, the first functional group is an amine group and the second functional group is a carboxyl group. Alternatively, the first functional group is a carboxyl group and the second functional group is an amine group. Therefore the first functional group and the second functional group can form a peptide bond therebetween.

The amine group, in the context of the first and/or second functional group, can originate from an N-alpha amine of an amino acid residue, or from an amine on the side-chain of an amino acid residue, such as found for example, in lysine and ornithine. Alternatively, the amine can stem from a hydrophobic moiety residue, such as, for example, an amino-fatty acid. Similarly, the carboxyl group, in the context of the first and/or second functional group, can originate from a C-alpha carboxyl of an amino acid residue, or from a carboxyl on the side-chain of an amino acid residue, such as found for example, in aspartic acid and glutamic acid. Alternatively, the amine can stem from a hydrophobic moiety residue, such as, for example, an amino-fatty acid. Similarly, the carboxyl group can stem from a hydrophobic moiety residue, such as, for example, any fatty acid.

Preferably, the one of the first or second functional groups is an amine on a hydrophobic moiety residue, and the other functional group is a carboxyl on an amino acid residue.

As used herein, the term "amine" describes a -NR'R" group where each of R' and R" is independently hydrogen, alkyl, cycloalkyl, heteroalicyclic, aryl or heteroaryl, as these terms are defined herein.

As used herein, the term "alkyl" describes an aliphatic hydrocarbon including straight chain and branched chain groups. Preferably, the alkyl group has 1 to 20 carbon atoms, and more preferably 1-10 carbon atoms. Whenever a numerical range; e.g., "1-10", is stated herein, it implies that the group, in this case the alkyl group, may contain 1 carbon atom, 2 carbon atoms, 3 carbon atoms, etc., up to and including 10 carbon atoms. The alkyl can be substituted or unsubstituted. When substituted, the substituent can be, for example, an alkyl, an alkenyl, an alkynyl, a cycloalkyl, an aryl, a heteroaryl, a halide, a hydroxy, an alkoxy and a hydroxyalkyl as these terms are defined hereinbelow. The term "alkyl", as used herein, also encompasses saturated or unsaturated hydrocarbon, hence this term further encompasses alkenyl and alkynyl.

The term "alkenyl" describes an unsaturated alkyl, as defined herein, having at least two carbon atoms and at least one carbon-carbon double bond. The alkenyl may be substituted or unsubstituted by one or more substituents, as described hereinabove.

The term "alkynyl", as defined herein, is an unsaturated alkyl having at least two carbon atoms and at least one carbon-carbon triple bond. The alkynyl may be substituted or unsubstituted by one or more substituents, as described hereinabove.

The term "carboxyl", as used herein, refers to a -C(=O)-O-R', where R' is as defined herein. When R' is hydrogen the carboxyl group is referred to as a carboxylic acid, and when R' is an alkyl, the carboxyl group is referred to as an ester.

The term "amide" describes a -NR'-C(=O)- group, a -NR'-C(=O)-R" group or a -C(=O)-NR'R" group, wherein R' is as defined herein and R" is as defined for R'. An amide is used herein interchangeably with peptide bond.

The term "hydroxyl", as used herein, refers to an -OH group.

As used herein, the term "aldehyde" refers to a -C(=O)H group.

The term "imine", which is also referred to in the art interchangeably as "Schiff-base", describes a -N=CR'- group, with R' as defined herein. As is well known in the art, Schiff bases are typically formed by reacting an aldehyde and an amine-containing moiety such as amine, hydrazine, hydrazide and the like, as these terms are defined herein.

Unless stated otherwise, the use of the terms "polymer" and "polymers" herein refers to both the cyclic and/or the linear form thereof.

The polymer, according to the present embodiments, may have two or more hydrophobic moiety residues, whereby at least one is linked to one amino acid at one end and to another amino acid residue at another end, and another may elongate the polymeric chain by being linked to either one of the termini thereof, for example to the N-alpha of a terminal amino acid residue and/or the C-alpha of a terminal amino acid residue. Optionally, a second hydrophobic moiety may be linked to a side-chain of an amino acid residue in the polymer.

The net positive charge of the polymer, which is one of the key characteristics of AMPs which were found to be linked to their activity, is maintained by having one or more positively charged amino acid residues in the polymer, optionally in addition to the positively charged N-terminus amine.

As used herein the phrase "positively charged amino acid" describes a hydrophilic amino acid with a side chain pKa value of greater than 7, namely a basic amino acid. Basic amino acids typically have positively charged side chains at physiological pH due to association with a hydronium ion. Naturally occurring (genetically encoded) basic amino acids include lysine (Lys, K), arginine (Arg, R) and histidine (His, H), while non-natural (non-genetically encoded, or non-standard) basic amino acids include, for example, ornithine, 2,3,-diaminopropionic acid, 2,4-diaminobutyric acid, 2,5,6-triaminohexanoic acid, 2-amino-4-guanidinobutanoic acid, and homoarginine.

In one embodiment of the present invention, all the amino acid residues in the polymer are positively charged amino acid residues. Exemplary polymers according to this embodiment include a plurality of lysine residues.

In one embodiment of the present invention, each of the components in the polymer according to the present embodiments is preferably linked to the other by a peptide bond.

The terms "peptide bond" and "amide bond" as used herein refer to an amide group, namely, a -(C=O)NH- group, which is typically formed by nucleophilic addition-elimination reaction between a carboxylic group and an amine group, as these terms are defined herein.

However, the polymers of the present embodiments may have other bonds linking the various components in the polymeric structure. Such non-peptidic bonds may render the polymer more stable while in a body or more capable of penetrating into cells. Thus, peptide bonds (-(C=O)NH-) within the polymer may be replaced, for example, by N-methylated amide bonds (-(C=O)NCH₃-), ester bonds (-C(R)H-C(=O)-O-C(R)-N-), ketomethylen bonds (-C(=O)CH₂-), aza bonds (-NH-N(R)-C(=O)-), wherein R is any alkyl, e.g., methyl, carba bonds (-CH₂-NH-), hydroxyethylene bonds (-CH(OH)-CH₂-), thioamide bonds (-CS-NH-), olefinic double bonds (-CH=CH-), retro amide bonds (-NH-(C=O)-), peptide derivatives (-N(R)-CH₂-C(=O)-), wherein R is the "normal" side chain, naturally presented on the carbon atom. These modifications can occur at any of the bonds along the polymer chain and even several (2-3) at the same time.

In one embodiment, all of the bonds in the polymer, linking the amino acid residues and hydrophobic moiety residues to each other, are peptide bonds. For example, in one embodiment, the polymer is made of an amino acid residue linked by a peptide bond to a hydrophobic moiety residue which in turn is linked to a second amino acid residue by another peptide bond. In another example, the polymer of the previous example is elongated by a second hydrophobic moiety residue which is linked to any one of the N- or C- termini by a peptide bond, etcetera.

The polymer, according to some embodiments, includes from 2 to 50 amino acid residues. More preferably, the polymer includes from 2 to 8 amino acid residues and more preferably from 2 to 6 amino acid residues.

The polymer, according to some embodiments, includes from 1 to 50 hydrophobic moiety residues. More preferably, the polymer comprises from 1 to 12 hydrophobic moiety residues, more preferably from 1 to 8 hydrophobic moiety residues and more preferably from 1 to 6 hydrophobic moiety residues.

The hydrophobic moieties that are used in the context of this and other preferred embodiments have one or more hydrocarbon chains, and are capable of linking to one or two other components in the polymer (e.g., one or two of an amino acid residue and another hydrophobic moiety) via two peptide bonds. These moieties therefore preferably have a carboxylic group at one end of the hydrocarbon chain (for linking a free amine group) and an amine group at the other (for linking a carboxylic acid group).

The hydrocarbon chain connecting the carboxylic and amine groups in such a hydrophobic moiety preferably has from 4 to 30 carbon atoms.

In one embodiment of the present invention, the hydrophobic moiety residue is a fatty acid residue wherein the hydrocarbon chain can be unbranched and saturated, branched and saturated, unbranched and unsaturated or branched and unsaturated. More preferably the hydrocarbon chain of the fatty acid residue is an unbranched and saturated chain having from 4 to 30 carbon atoms, preferably from 4 to 20 carbon atoms. Non-limiting example of such fatty acid residues are butyric acid residue, such as γ-aminobutyric acid residue and α-aminobutyric acid residue, caprylic acid residue, lauric acid residue, palmitoleic acid residue and oleic acid residue.

In one embodiment, the fatty acid residue has an amine on the distal carbon of the hydrocarbon chain (with respect to the carboxylic acid group). Such a fatty acid residue is referred to herein as a ω-amino fatty acid residue. Again here the hydrocarbon chain of the ω-amino fatty acid residue may have from 4 to 30 carbon atoms.

The term "ω-amino-fatty acid" refers to linear amino fatty acids which have an amino group at the end-carbon thereof. Exemplary ω-amino-fatty acids include, without limitation, 4-amino-butyric acid, 6-amino-caproic acid, 8-amino-caprylic acid, 10-amino-capric acid, 12-amino-lauric acid, 14-amino-myristic acid, 16-amino-palmitic acid, 18-amino-stearic acid, 18-amino-oleic acid, 16-amino-palmitoleic acid, 18-amino-linoleic acid, 18-amino-linolenic acid and 20-amino-arachidonic acid 4-amino-butyric acid, 6-amino-caproic acid, 8-amino-caprylic acid, 10-amino-capric acid, 12-amino-lauric acid, 14-amino-myristic acid, 16-amino-palmitic acid, 18-amino-stearic acid, 18-amino-oleic acid, 16-amino-palmitoleic acid, 18-amino-linoleic acid, 18-amino-linolenic acid and 20-amino-arachidonic acid

According to some embodiments of the present invention, the hydrophobic moiety is selected from the group consisting of 4-amino-butyric acid, 8-amino-caprylic acid and 12-amino-lauric acid and more preferably is 8-amino-caprylic acid and 12-amino-lauric acid.

The linear polymers described herein can be represented collectively by the following general Formula I:

X-W₀-[A₁-Z₁-D₁]-W₁-[A₂-Z₂-D₂]-W₂- ... [An-Zn-Dn]-Wn-Y Formula I

wherein:
n is an integer from 2 to 50, preferably from 2 to 12 and more preferably from 2 to 8;
A₁, A₂, ..., An are each independently an amino acid residue, preferably a positively charged amino acid residue, more preferably all of A₁, A₂, ..., An are positively charged amino acid residues as discussed hereinabove, such as histidine residues, lysine residues, ornithine residues and arginine residues ,and most preferably all the positively charged amino acid residues are lysine residues;
D₁, D₂, ..., Dn are each independently a hydrophobic moiety residue, as defined and discussed hereinabove, or absent, provided that at least one such hydrophobic moiety residue exists in the polymer, preferably at least one of the hydrophobic moiety residues is a ω-amino-fatty acid residue;
Connecting each monomer of the residue are linking moieties, denoted Z₁, Z₂, ..., Zn and W₀, W₁, W₂, ..., Wn, each of which independently linking an amino acid residue and a hydrophobic moiety residue or absent, preferably at least one of the linking moieties is a peptide bond and most preferable all the linking moieties are peptide bonds;

The fringes of the polymer, denoted X and Y, may each independently be hydrogen, an amine, an amino acid residue, a hydrophobic moiety residue, is another polymer having the general Formula I or absent.

Exemplary linear polymers according to the present embodiments are those having the structures presented here in below : 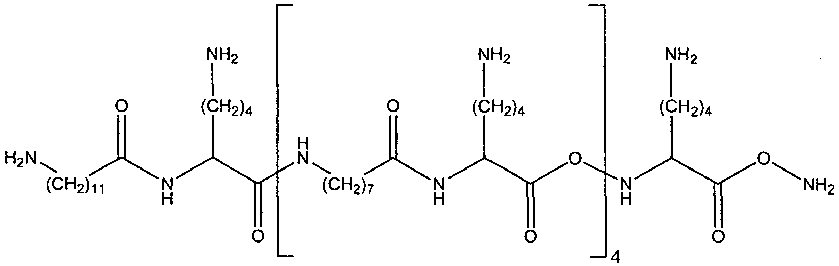 which is also referred to herein as NC₁₂K(C₈K)₄KNH₂; and 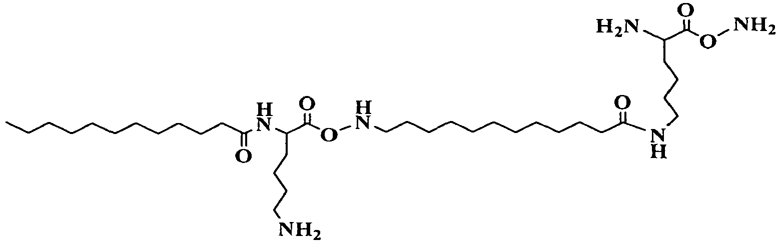 which is also referred to herein as C₁₂KNC₁₂K(ε)NH₂.

Other exemplary linear polymers are presented in U.S. Patent Application Nos. 11/234,183 and 11/500,461 and WO 2006/035431.

The cyclic polymers described herein can be represented collectively by the following general Formula II: 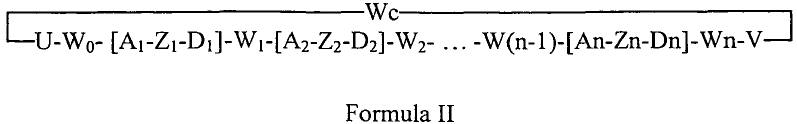 wherein:
n is an integer from 2 to 50, preferably from 2 to 12 and more preferably from 2 to 8;
A₁, A₂, ..., An are each independently an amino acid residue, preferably a positively charged amino acid residue, more preferably all of A₁, A₂, ..., An are positively charged amino acid residues as discussed hereinabove, such as histidine residues, lysine residues, ornithine residues and arginine residues ,and most preferably all the positively charged amino acid residues are lysine residues;
D₁, D₂, ..., Dn are each independently a hydrophobic moiety residue, as defined and discussed hereinabove, or absent, provided that at least one such hydrophobic moiety residue exists in the polymer, preferably at least one of the hydrophobic moiety residues is a ω-amino-fatty acid residue;
Connecting each monomer of the residue are linking moieties, denoted Z₁, Z₂, ..., Zn and W₁, W₂, ..., Wn-1, each of which independently linking an amino acid residue and a hydrophobic moiety residue or absent.

U is selected from the group consisting of the first functional group, as defined hereinabove, an amino acid residue having that first functional group, a hydrophobic moiety residue having that first functional group, and a linking moiety having that first functional group, or absent.

Similarly, V is selected from the group consisting of the second functional group, an amino acid residue having that second functional group, a hydrophobic moiety residue having that second functional group, and a linking moiety having that second functional group, or absent.

The linking moiety W₀ is linking any one of A₁, Z₁ and D₁ to U, or absent, and the linking moiety Wn is linking any one of An, Zn and Dn to V, or absent;

Wc is a cyclizing moiety.

The moieties which close the polymer into a cyclic polymer, denoted U and V, may each independently be absent or be an amino acid residue or a hydrophobic moiety residue, provided they each has a functional group, referred to hereinabove as the first and second functional groups, which can form a covalent bond therebetween. Thus, such amino acid residues and/or hydrophobic moiety residues can form together a unique linking moiety denoted herein as Wc, which is referred to herein as the cyclizing moiety.

As used herein, the phrase "linking moiety" describes a chemical moiety, group or a bond, as defined herein, which links between two residues or monomers. The linking moiety can thus be, for example, formed upon reacting two functional groups; each forms a part of another monomer or residue, thus linking the two monomers or residues. For example, an amine group on one monomer can form a peptide bond with a carboxyl group on another monomer and the resulting moiety is a peptide bond linking moiety.

Preferably, at least one of the linking moieties in the polymers presented herein is a peptide bond, and most preferable all the linking moieties are peptide bonds.

The phrase "cyclizing moiety", denoted Wc in Formula II, refers to a chemical moiety which is formed when two residues in Formula II are linked therebetween, thereby forming the cyclic polymer. The cyclizing moiety may be, for example, a bond which is formed between two functional groups, such as, for a non-limiting example, an amide (peptide bond), a carboxylate (ester), a carbamate, an ether and the likes.

The two functional groups which form Wc, can stem from U and V, W₀ and Wn, or A₁, Z₁ and D₁ and An, Zn or Dn, or any combination thereof. Alternatively, the cyclizing moiety may comprise a residue of a multifunctional (as at least bifunctional) moiety which forms bonds with functional groups on U and V, W₀ and Wn, or A₁, Z₁ and D₁ and An, Zn or Dn, such as, for a non-limiting example, p-aminobenzoic acid or ethyleneglycol.

Preferably the cyclizing moiety, denoted Wc, is a peptide bond which is formed from an amine group on either U of V, and a carboxyl on either V or U.

Hence, for better clarity, the phrase "cyclic polymer" as used herein in the context of the polymer, refers to a polymer that comprises an intramolecular covalent bond which forms a part of a cyclizing moiety. The cyclizing moiety is positioned between two non-adjacent residues therein, forming a cyclic polymer ring that comprises at least two amino acid residues, at least one hydrophobic moiety residue, a cyclizing moiety and optionally further comprise a plurality of linking moieties and other residues. The cyclizing moiety may connect backbone to any two residues in the polymer via backbone atoms, side-chain atoms or a combination thereof.

Preferred cyclic polymers are polymers in which n is an integer from 2 to 5, the amino acid residues are all lysine residues, and the hydrophobic moiety residues are all 12-amino-lauric acid residues.

Exemplary cyclic polymers according to the present embodiments are those having the structures presented hereinbelow: 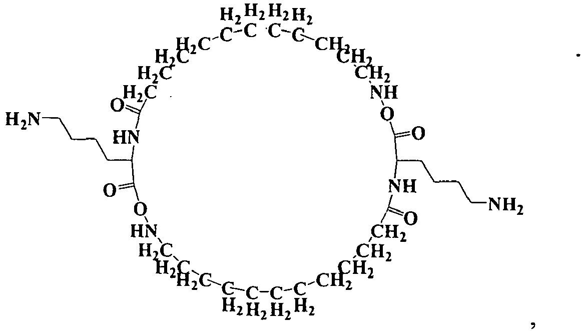 which is also referred to herein as Cyclic-(NC₁₂K)₂; and 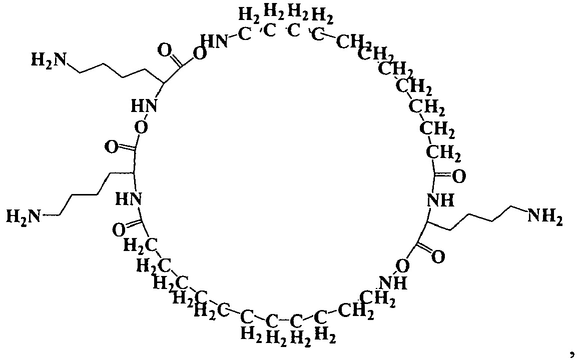 which is also referred to herein as Cyclic-NC₁₂KKNC₁₂K.

As discussed above, one or more of the hydrophobic moiety residues may be attached to a side chain of one or more of the amino acid residues of the polymer, i.e., act as a branch of the main linear or cyclic polymer.

The anticancerous polymers according to the present embodiments can be readily synthesized as demonstrated for structurally similar antimicrobial polymers in U.S. Patent Application Nos. 11/234,183 and 11/500,461 and WO 2006/035431, in U.S. Provisional Patent Application, by the present assignee, having Attorney's Docket No. 38146 and entitled "Novel Antimicrobial Agents", which is co-filed with the instant application, and in the Examples section that follows hereinbelow. For example, polymers in which the linking moieties are peptide bonds, and hence resemble natural and synthetic peptides in this respect, can be prepared by classical methods known in the art for peptide syntheses. Such methods include, for example, standard solid phase techniques. The standard methods include exclusive solid phase synthesis, partial solid phase synthesis methods, fragment condensation, classical solution synthesis, and even by recombinant DNA technology. See, e.g., Merrifield, J Am. Chem. Soc., 85:2149 (1963), incorporated herein by reference. Solid phase peptide synthesis procedures are well known in the art and further described by John Morrow Stewart and Janis Dillaha Young, Solid Phase Peptide Syntheses (2nd Ed., Pierce Chemical Company, 1984).

The anticancerous polymers of the present embodiments can be purified, for example, by preparative high performance liquid chromatography [Creighton T. (1983) Proteins, structures and molecular principles. WH Freeman and Co. N.Y.].

The anticancerous polymers of the present invention are selected, designed and utilized to destroy neoplastic tissues by killing cancerous cells selectively and destructing at least a portion of the neoplastic tissue. Hence, according to the present embodiments, the polymer is having an anticancerous activity *per se,* as well as other biochemical and biological activities based on their unique capacities to interact with living cells such as cancerous cells.

As well acknowledged in the art, disrupting the outer membrane of a cell causes its death due to membrane depolarization, leakage of metabolites and/or total loss of cell integrity. Without being bound by any particular theory, membrane disruption is one of the postulated activities attributed to the polymers presented herein; therefore the polymers of the present invention may act directly as effective anticancerous agents by disrupting the metabolism and/or the multiplication processes of the neoplastic tissue by disrupting the outer membrane thereof. More specifically, yet without being bound to any particular theory, the anticancerous activity of the polymers presented herein can be expressed by partially or entirely disrupting any membrane of at least a portion of the cells of a neoplastic tissue, including sub-cellular components thereof such as the mitochondria.

The phrase "anticancerous activity" as used herein refers to a therapeutic activity of a substance which can be used to treat cancer by directly or indirectly destroying neoplastic cells and tissues selectively with respect to benign cells and tissues.

The anticancerous polymers presented herein can be used to treat a wide spectrum of cancers (neoplasms), such as carcinoma, lymphoma, leukemia, sarcoma, mesothelioma, glioma, germinoma, choriocarcinoma, and any other neoplastic tissue.

The phrase "neoplastic tissue" as used herein, refers to an abnormal, disorganized and typically uncontrolled proliferation and growth of cells in a tissue or an organ, usually forming a distinct mass of cells which is commonly referred to as a growth, neoplasm or tumor, and collectively referred to as cancer.

The polymers presented herein are designed so as to have an anticancerous activity also in cases where the cancer is known as multidrug resistance (MDR), as discussed hereinabove, regardless if the MDR is inherent to a particular cancerous tissue it is applied against, or if the MDR is acquired.

Thus, the polymers presented herein can be beneficially used also in cases where the cancer tissues are able to develop multidrug resistance or prone to develop multidrug resistance, or turned into a multidrug resistant cancer in a course of any conventional treatment, or immerged as a multidrug resistant cancer.

As is discussed in detail hereinabove, MDR is effected via an extrusion mechanism, which involves energy-dependant extrusion channels or pumps that actively pump the drug out of the cells, thereby reducing its intracellular concentration below lethal threshold. Chemosensitizers, in this respect, are agents that reverse or modulate multidrug resistance in MDR cells by modulating the activity of the MDR extrusion pumps.

Without being bound to any particular theory, it is possible that the polymers presented herein act as chemosensitizers, sensitizing MDR cancer cells to a cytotoxic agent.

As demonstrated in the Examples section that follows, exemplary polymers according to the present embodiments have been shown to exhibit cytotoxic activity which is selective towards cancerous cells, such as human breast adenocarcinoma cells, variant small cell lung cancer cells and transgenic adenocarcinoma mouse prostate cells, over benign cells such as rat cardiac fibroblasts, MDR induced CEM T-cells, C6 glioma cells and human red blood cells.

Apart from having beneficial anticancerous activity *per se,* as detailed herein, the polymers presented herein may include one or more additional active agents attached thereto which assist in the treatment of cancer, such as labeling, targeting and chemosensitization of cancerous cells and neoplastic tissues. The conjugation of the additional active agent(s) to the polymers presented herein can provide a dual utility for the polymer. Furthermore, the option to include an additional active agent may produce a synergistic effect, enhancing the anticancerous activity of the polymer as well as the activity of the additional active agent.

According to some embodiments of the present invention, the additional active agent may be attached to the polymer at any substitutable position. Examples of such substitutable positions include, without limitation, a side chain of any one or more of the amino acid residues in the polymer, any one of the linking moieties of the polymer, any one of the N- and C- termini of the polymer and any one or more of the hydrophobic moiety residues in the polymer.

As used herein, the phrase "active agent" refers to a compound or a portion of a compound, which exhibits a pharmacological or biological beneficial activity *per se.* Examples include a therapeutically active agent, a chemosensitization agent, a targeting agent, a labeling agent (such as for imaging and diagnostic purposes) and the likes. According to embodiments of the present invention, a polymer can have more than one active agents attached thereto, and more than one type of active agents attached thereto.

As used herein, the phrase "therapeutically active agent" describes a chemical substance, which exhibits a therapeutic activity when administered to a subject.

Non-limiting examples of general therapeutically active agents that can be beneficially used in this and other contexts of the present invention include, without limitation, an anticancerous agent, an anti-proliferative drug, chemotherapeutic drug, an agonist, an amino acid, an analgesic, an antagonist, an antibiotic agent, an antibody, an antidepressant agent, an antigen, an anti-histamine, an anti-hypertensive agent, an anti-inflammatory drug, an anti-metabolic agent, an antimicrobial agent, an antioxidant, an antisense, a co-factor, a cytokine, a drug, an enzyme, a growth factor, a heparin, a hormone, an immunoglobulin, an inhibitor, a ligand, a nucleic acid, an oligonucleotide, a peptide, a phospholipid, a prostaglandin, a protein, a toxin, a vitamin and any combination thereof.

The combined therapeutic effect is particularly advantageous when the therapeutically active agent is an anticancerous agent. The term "anticancerous agent" refers to an agent which exhibits anticancerous activity, and encompasses chemotherapeutic agents, anti-proliferative agents and radiotherapeutic, which are agents that, when contacted with and/or incorporated into a cell, produce an effect on the cell including causing the death of the cell, inhibiting cell division or inducing differentiation. The combined activity of the anticancerous polymers of the present invention and that of an additional anticancerous agent may provide the additional anticancerous agent the capacity to overcome the known limitations of these agents, as well as evoke a synergistic effect.

As used herein, the term "radiotherapeutic" refers to a certain type of anticancerous agents comprising ionizing radiation producing radionuclides (radioactive isotopes or radioisotopes), which when contacted with and/or incorporated into a cell, kill the cell by means of the ionizing radiation.

The phrase "synergistic effect" or "synergism" as used herein refers to the phenomenon in which two or more discrete activities or agents acting together create an effect greater than the predicted sum of the separate effects of the individual agents.

Non-limiting examples of anticancerous agents include, without limitation, aminoglutethimide, amsacrine, azacitidine, aziridine, bleomycin, busulfan, capecitabine (also known as Xeloda), carboplatin, carmustine, chlorambucil, cisplatin, cyclophosphamide, cytarabine, cytoxan, dacarbazine, dactinomycin, daunorubicin, 4'-deoxydoxorubicin, dexamethasone, diethylstil-bestrol, docetaxel, doxorubicin, estramustine, ethinyl estradiol, etoposide, finasteride, floxuridine, fludarabine, 5-fluoruracil, fluoxymesterone, flutamide, fluxoridine, gemcitabine, goserelin, hexamethyl-melamine, hydroxy- progesterone caproate, hydroxyurea, ifosfamide, interferon alfa, irinotecan, L-asparaginase, leuprolide, lomustine, mechlorethamine, medroxy-progesterone acetate, megestrol acetate, melphalan, 6-mercaptopurine, methotrexate, mitomycin, mitotane, mitoxantrone, nitrosourea, oxaliplatin, paclitaxel, pentostatin, plicamycin, procarbazine, propionate, semustine, streptozocin, tamoxifen, tegafur, temozolomide, teniposide, teniposide, testosterone, thalidomide, thioguanine, thiotepa, topotecan, trimetrexate, tumor infiltrating lymphocytes, tumor necrosis factor, vinblastine (VLB), vinca alkaloid, vincristine, vindesine, vinorelbine, and any combination thereof.

According to some embodiments, the anticancerous polymers presented herein may have an additional chemosensitization agent (chemosensitizer) attached thereto.

As used herein, the term "chemosensitizer" refers to a substance which can increase the efficacy of a therapeutic agent against a multidrug resistant cell and/or decrease the resistance of an MDR cell for a therapeutic agent. For example, a chemosensitizer can inhibit the functioning of a particular cellular glycoprotein and thereby make cells, especially tumor cells, sensitive to anticancerous and chemotherapeutic agents. Accordingly, the term "chemosensitization" means an apparent increase or an apparent enhancement of the measured cytotoxicity of an therapeutic agent on multidrug resistance cells in the presence of a chemosensitizer, as is compared to the level of cytotoxicity exerted by the therapeutic agent in the absence of the chemosensitizer.

The terms "chemosensitizer" and "chemosensitization" are meant to encompass the aspects of nuclear medicine, and in particular treatment with radiotherapeutic agents and direct irradiation of cancerous tissues in a subject, wherein radiosensitizing agents are used to increase the cytotoxic effect of the radioactivity.

The term "radiosensitizing" is meant to refer to agents that increase the susceptibility of cells to the damaging effects of ionizing radiation. In effect, a radiosensitizing agent permits lower doses of radiation to be administered and still provide a therapeutically effective dose.

Representative chemosensitizers useful in the context of the present embodiments include, but are not limited to a calcium channel blocker, a calmodulin inhibitor, a cyclic peptide antibiotic, a cyclosporin analog, a detergent, an indole alkaloid, a lysosomotropic agent, a quinoline, a steroid, a triparanol analog, and more specifically 1,9-dideoxyforskolin, acridine, acridine orange, AHC-52 (ACS No. 119666-09-0), atropine, chinchonidine, chloroquine, corynanthine, cremophor EL, cyclosporin, desmethoxyverapamil, dihydrocyclosporin, fluoxetine, forskolin, GF-120918 (ACS No. 143664-11-3), MS-073 [Fukazawa et al., European Patent Application No. 0363212], MS-209 (5-[3-[4-(2,2-diphenylacetyl)piperazin-1-yl]2-hydroxypropoxy]quinoline sesquifumarate), physostigmine, primaquine, progesterone, propanolol, quinacrine, quinine, reserpine, RU-486 (17β-hydroxy-11β-[4-dimethylaminophenyl]-17α-prop-1-ynyl-estra-4,9-dien-3-one), RU-49953 (17β-hydroxy-11β,17α-[4-dimethylaminophenyl]-17α-prop-1-ynyl-estra-4,9-dien-3-one), S9778 (6-{4-[2,2-di-ethylamino]-1-piperidinyl}-N,N',di-2-propenyl-1,3,5-triazine-2,4-diamine-bismethane sulfonate), tacrolimus (also known as FK-506 or fujimycin), tamoxifen, trifluoperazine, trifluoroperazine chlorpromazine, tryptamine, verapamil, VX-710 (2-peperidinecarboxylic acid, 1-[oxo(3,4,5-trimethoxyphenyl)acetyl]-3-(3-pyridinyl)-1-[3(3-pyridinyl)propyl]butyl ester), VX-853 (ACS No. 190454-58-1), XR-9051 (ACS No. 57-22-7) and yohimbine (also known as quebrachin, aphrodin, corynine, yohimvetol or hydroergotocin).

As demonstrated in the Examples section that follows, the polymers presented herein have a specific affinity towards cancerous cells, yet this trait can be further enhanced by a cancerous cells and neoplastic tissues targeting agent. Hence, according to preferred embodiments, the anticancerous polymers presented herein may have an additional targeting agent attached thereto.

As used herein, the term "targeting agent" describes agents which have a specific affinity to cancerous cells and neoplastic tissues. Targeting agents may be used to deliver an anticancerous agent in general and a polymer according to the present invention in particular, to cancerous cells and tissues. The result is an enhanced effect and an improved exposure of the cancerous cells and neoplastic tissues to the anticancerous agent, preferably accompanied by reduced exposure of non-cancerous cells to the anticancerous agent.

Targeting agents include, for example, porphyrins, hormones, peptides, proteins, receptor ligands, antigens, haptens, antibodies and fragments thereof.

Labeling of cancerous growth is critical for the diagnosis and efficient targeting of the cancer and treatment thereof. Due to the aptitude of the polymers presented herein to bind to the cell membrane of cancerous cells of neoplastic tissues as discussed hereinabove, the polymers can be used of for labeling neoplastic tissues. The labeling can be effected by attaching one or more labeling agents to the polymer, and after administering thereof applying the appropriate detection technique. For example, when the labeling agent is an imaging agent, the appropriate detection technique is an imaging technique.

As used herein, the term "imaging agent" is meant to refer to agents which emit a detectable signal which can be traced to a particular position coordinates in the subject's body, wherein a full or partial signal detection scan can produce a set of coordinates that can be converted into an image showing the location(s) of the imaging agent(s) in the subject's body.

As used herein, the phrase "labeling agent" refers to a detectable moiety or a probe and includes, for example, chromophores, fluorescent agents, phosphorescent agents, heavy metal clusters, and radioactive labeling agents, as well as any other known detectable agents.

As used herein, the term "chromophore" refers to a chemical moiety that, when attached to another molecule, renders the latter colored and thus visible when various spectrophotometric measurements are applied.

The phrase "fluorescent agent" refers to a compound that emits light at a specific wavelength during exposure to radiation from an external source.

The phrase "phosphorescent agent" refers to a compound emitting light without appreciable heat or external excitation as by slow oxidation of phosphorous.

A heavy metal cluster can be for example a cluster of gold atoms used, for example, for labeling in electron microscopy techniques.

As presented in the Examples section that follows, the present inventors have successfully attached a D(+)-Glucosamine hydrochloride to the C-terminus of an exemplary polymer according to the present embodiments.

As also presented in the Examples section that follows, the present inventors have successfully attached a labeling agent to selected resin-bound polymers, which were labeled with the fluorescent probe 7-fluoro-4-nitrobenzo-2-oxo-1,3-diazole (NBD-F) following removal of the Fmoc protecting group from the N-terminal amino acid of the polymer.

The anticancerous polymers of the present invention can be utilized either *per se*, or as an active ingredient that forms a part of a pharmaceutical composition. Hence, according to another aspect of the present invention, there is provided a pharmaceutical composition which includes, as an active ingredient, one or more of the polymers presented herein and a pharmaceutically acceptable carrier. The composition is packaged in a packaging material and identified in print, in or on the packaging material, for use in the treatment of cancer.

Accordingly, the polymers presented herein can be used in the preparation of a medicament for the treatment of cancer.

As used herein a "pharmaceutical composition" refers to a preparation of the anticancerous polymer described herein, with other chemical components such as pharmaceutically acceptable and suitable carriers and excipients. The purpose of a pharmaceutical composition is to facilitate administration of a compound to a subject.

Hereinafter, the term "pharmaceutically acceptable carrier" refers to a carrier or a diluent that does not cause significant irritation to an organism and does not abrogate the biological activity and properties of the administered compound. Examples, without limitations, of carriers are: propylene glycol, saline, emulsions and mixtures of organic solvents with water, as well as solid (e.g., powdered) and gaseous carriers.

Herein the term "excipient" refers to an inert substance added to a pharmaceutical composition to further facilitate administration of a compound. Examples, without limitation, of excipients include calcium carbonate, calcium phosphate, various sugars and types of starch, cellulose derivatives, gelatin, vegetable oils and polyethylene glycols.

Techniques for formulation and administration of drugs may be found in "Remington's Pharmaceutical Sciences" Mack Publishing Co., Easton, PA, latest edition, which is incorporated herein by reference.

Pharmaceutical compositions for use in accordance with the present invention thus may be formulated in conventional manner using one or more pharmaceutically acceptable carriers comprising excipients and auxiliaries, which facilitate processing of the polymers into preparations which, can be used pharmaceutically. Proper formulation is dependent upon the route of administration chosen. Toxicity and therapeutic efficacy of the polymers described herein can be determined by standard pharmaceutical procedures in experimental animals, e.g., by determining the EC₅₀, the IC₅₀ and the LD₅₀ (lethal dose causing death in 50 % of the tested animals) for a subject polymer. The data obtained from these activity assays and animal studies can be used in formulating a range of dosage for use in human.

The dosage may vary depending upon the dosage form employed and the route of administration utilized. The exact formulation, route of administration and dosage can be chosen by the individual physician in view of the patient's condition. (See e.g., Fingl et al., 1975, in "The Pharmacological Basis of Therapeutics", Ch. 1 p.1).

The amount of a composition to be administered will, of course, be dependent on the subject being treated, the severity of the affliction, the manner of administration, the judgment of the prescribing physician, etc.

Compositions of the present invention may, if desired, be presented in a pack or dispenser device, such as an FDA (the U.S. Food and Drug Administration) approved kit, which may contain one or more unit dosage forms containing the active ingredient. The pack may, for example, comprise metal or plastic foil, such as, but not limited to a blister pack or a pressurized container (for inhalation). The pack or dispenser device may be accompanied by instructions for administration. The pack or dispenser may also be accompanied by a notice associated with the container in a form prescribed by a governmental agency regulating the manufacture, use or sale of pharmaceuticals, which notice is reflective of approval by the agency of the form of the compositions for human or veterinary administration. Such notice, for example, may be of labeling approved by the U.S. Food and Drug Administration for prescription drugs or of an approved product insert. Compositions comprising a polymer according to preferred embodiments are formulated in a compatible pharmaceutical carrier may also be prepared, placed in an appropriate container, and labeled for treatment of an indicated condition or diagnosis, as is detailed hereinabove.

Due to their membrane disrupting character, the polymers described herein can act synergistically with another active agent by permeabilizing the cancerous cells of the neoplastic tissue. This permeabilizing action of the polymers can increase the uptake of other therapeutically active agents and therefore should be able to potentiate their activity.

The use of the polymers presented herein in any of the aspects presented above, namely the method of cancer treatment, the use in the preparation of a medicament and the pharmaceutical composition for the treatment of cancer, in combination with another therapeutically active agent, can improve the effect of both the polymer and the therapeutically active agent, and preferably create a synergistic effect, particularly when the therapeutically active agent is an anticancerous agent and/or a chemosensitization agent.

Hence, according to some embodiments of the present invention, the method of treatment may include the administration of an additional therapeutically active agent, and preferably an anticancerous agent and/or a chemosensitization agent.

Accordingly, the pharmaceutical composition as well as the medicament presented hereinabove, may further comprise at least one additional therapeutically active agent, and preferably the therapeutically active agent is an anticancerous agent and/or a chemosensitization agent.

In order to allow their combined action by their dual presence in the treated cell, and maximize the combined effect, it is advantageous that the chemosensitization agent or the anticancerous agent would be administered substantially at the same time with the anticancerous polymer presented herein.

The administration of a chemosensitization agent and the anticancerous polymer substantially at the same time is a highly important and advantageous feature in the treatment of multidrug resistance (MDR) cells.

Hence, the phrase "substantially at the same time", as used herein, means that the anticancerous polymer and the chemosensitization agent are administered in such time intervals that would allow their dual presence in effective concentrations in the treated cells. The anticancerous polymer and the chemosensitization agent can be administered by different or identical routes of administration.

Additional objects, advantages, and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following examples.

### EXAMPLES

Reference is now made to the following examples, which together with the above descriptions; illustrate the invention in a non limiting fashion.

### MATERIALS AND EXPERIMENTAL METHODS

### Polymer screening library - Chemical syntheses and analysis:

The polymers were produced by the solid phase method following methodologies disclosed in U.S. Patent Application Nos. 11/234,183 and 11/500,461 and WO 2006/035431.

Briefly, the polymers were synthesized while applying the Fmoc active ester chemistry on a fully automated, programmable peptide synthesizer (Applied Biosystems 433A). After cleavage from the resin, the crude product was extracted with 30 % acetonitrile in water and purified by RP-HPLC (Alliance Waters), so as to obtain a chromatographic homogeneity higher than 95 %. HPLC runs were typically performed on C₁₈ columns (Vydac, 250mm x 4.6 or 10mm) using a linear gradient of acetonitrile in water (1 % per minute), both solvents containing 0.1 % trifluoroacetic acid. The purified polymers were subjected to mass spectrometry (ZQ Waters) and NMR analyses to confirm their composition and stored as a lyophilized powder at -20 °C. Prior to being tested, fresh solutions were prepared in water, vortexed, sonicated, centrifuged and then diluted in the appropriate medium.

Selected resin-bound polymers were labeled with the fluorescent probe 7-fluoro-4-nitrobenzo-2-oxo-1,3-diazole (NBD-F) following removal of the Fmoc protecting group from the N-terminal amino acid of the polymer.

### Cell cultures:

In order to evaluate the cancer-cell killing capacity of the polymers against a variety of cancer types, and in order to evaluate their selectivity towards cancerous cells, meaning the ability to affect more than one type of cancerous cells while not affecting benign cells, the following cell cultures were used:
Human breast adenocarcinoma cells (MCF-7);
Variant small cell lung cancer cells (N-417, SCLC);
Transgenic adenocarcinoma mouse prostate cells (TRAMP-C2);
Primary non-cancerous rat cardiac fibroblasts (CF);
Human red blood cells (RBC);
Human CEM T-cell line is MDR; and
Rat C6 glioma cell line.

Cells were cultured in DMEM, RPMI-1640 supplemented with fetal calf serum and other necessary key components. The cultures were maintained in humidified 5 % CO₂ atmosphere at 37 °C, as recommended by the American type culture collection.

### Lethal and lytic concentration determination:

As a routine cancer cell killing assay for assessment of antitumor activity, 2,3-bis(2-methoxy-4-nitro-5-sulfophenyl)-2H-tetrazolium-5-carboxanilide inner salt (XTT) reduction assay was used, utilizing a commercial kit such as, for example, Cell Proliferation XTT Kit, Cat. No. 20-300-1000, Biological Industries Ltd., Kibbutz Beit Haemek, Israel. XTT is a tetrazolium salt which is cleaved to formazan colored compounds by the mitochondrial dehydrogenase enzymes indicating proper mitochondrial activity in intact cells.

Cells were seeded onto 96-well plates and incubated at humidified 5 % CO₂ atmosphere at 37 °C for 24 hours. Thereafter, a series of increasing concentrations of the polymers were added. After another 24 hours of incubation, XTT reaction solution was added (50 µl of XTT per 100 µl of cell culture medium). The optical density of the cells was measured at 450 nm by ELISA plate reader after 2-4 hours XTT incubation. Cell viability was determined relative to a control having no polymer added thereto. The LC₅₀ value for each polymer was estimated from the curve of cell viability versus polymer concentration and taken from the concentration at which cell viability was 50 %.

### Hemocytotoxicity:

Hemolytic activity was determined according to Antibacterial Peptides Protocols as presented by Tossi, A. et al. in Methods Mol. Biol., 1997, 78, pp. 133-150. The polymer's toxic potential was determined against human red blood cells (RBC) by measuring leakage of hemoglobin therefrom in phosphate buffer solution (PBS). Human blood samples were rinsed three times in PBS by centrifugation for 2 minutes at 200 x g, and re-suspended in PBS at 5 % hematocrit. A 50 µl-fractions of a suspension containing 2.5 x 10⁸ RBC were added to test tubes containing 200 µl of polymer solutions (2-fold serial dilutions in PBS), PBS alone (for base-line values), or distilled water (for 100 % hemolysis). After 3 hours incubation at 37 °C under agitation, samples were centrifuged, and hemolytic activity was determined as a function of hemoglobin leakage by measuring absorbance at 405 nm of 200 µl aliquots of the supernatants. Controls for base-line hemolysis (blank) and 100 % hemolysis consisted of RBC suspended in PBS and TritonX100 (0.2 %), respectively.

### Kinetic cytotoxicity studies:

The kinetic rate of the polymers' cytotoxic activity was monitored by using the XTT assay as described hereinabove for the cell viability assay, with some modifications, and compared to the typically used exemplary chemotherapy drugs mitomycin C and doxorubicin.

The cells were treated with a polymer solution in cell medium at a concentration equal to eight multiples of the LC₅₀ value thereof to ensure 100 % cells death. Thereafter the medium was replaced with a fresh solution and XTT was added at various time intervals of exposure to the peptides, namely 0, 15, 30, 60, 120 and 180 minutes.

### Cellular localization:

In order to visualize sub-cellular location of the polymers, the cells were seeded in a chamber cover glass and grown for 24 hours in an appropriate medium. To test mitochondrial localization of the polymers, the cells were washed and then pre-incubated with 7-fluoro-4-nitrobenzo-2-oxo-1,3-diazole (NBD-F), which forms a covalent linkage to the polymer's N-terminus, followed by incubation of the cells with the labeled polymers and incubation of the cells with the mitochondrial specific marker MitoTracker Red (Invitrogen). After washing and fixating with 4 % formaldehyde, the cells were examined and analyzed by confocal microscopy.

To test the localization of the polymers in the ER or Golgi apparatus, the cells were incubated with NBD-F-labeled polymers and were thereafter washed, fixed with 4 % formaldehyde and permeabilized with TritonX100 (0.1 %). Finally, the cells were blocked with donkey serum (10 % in PBS), incubated with the primary antibody anti-PDI for ER, and with anti-human golgin-97 for Golgi apparatus, followed by incubation with the appropriate secondary antibody. Unfixed or 4 % formaldehyde fixed cells were imaged using a confocal laser scanning microscope.

### EXPERIMENTAL RESULTS

### Polymer screening library:

Several representative series of polymers according to the present embodiments, substantially consisting of a plurality of lysine residues, and ω-amino-fatty acid residues and fatty acid residues as hydrophobic moieties, were prepared according to the general procedure described in U.S. Patent Application Nos. 11/234,183 and 11/500,461 and WO 2006/035431, each being incorporated herein in its entirety. These polymers are presented in Table 3 below.

These exemplary polymers are referred to in this section according to the following formula:

T[NCᵢK(x)]ⱼG or Cyclic-T[NCᵢK(x)]ⱼG

In this formula, the prefix "Cyclic-" denotes a cyclic polymer; NCᵢ denotes an ω-amino-fatty acid residue (an exemplary hydrophobic moiety according to the present invention, represented by D₁ ...Dn in the general formulae I and II described herein), whereby i denotes the number of carbon atoms in the fatty acid residue; K denotes a lysine residue (an exemplary amino acid residue according to the present invention, denoted as A₁...An in the general Formulae I and II described herein, such that [NCᵢK(x)] denotes a residue of an ω-amino-fatty acid-lysine conjugate (denoted as [A₁-Z₁-D₁] .... [An-Zn-Dn] in the general Formulae I and II described herein) wherein (x) denotes the type of amine group in the amino acid used for conjugation with one end of the hydrophobic moiety (e.g., the ω-amino-fatty acid), whereby when the denotation (x) is absent, it is meant that conjugation is effected via the N-alpha of the lysine residue and when (x) is (ε) it is meant that conjugation is effected via the epsilon amine of the lysine residue; j denotes the number of the repeating units of a specific conjugate in the polymer (corresponding to n in the general Formulae I and II described herein); and T and G each independently denotes either a hydrogen (no denotation), a lysine residue (denoted K), an ω-amino-fatty acid residue (denoted NCᵢ), a fatty acid residue (denoted Cᵢ), an ω-amino-fatty acid-lysine conjugate residue (denoted NCᵢK), a fluorenylmethyloxycarbonyl residue (denoted Fmoc), a benzyl residue (denoted Bz), a cholate residue (denoted Chl), an amine group (typically forming an amide at the C-terminus and denoted NH₂), and free acid residue (for the C-terminus no denotation), an alcohol residue, and any combination thereof (all corresponding to X and Y in the general formula I described herein).

Thus, for example, a polymer according to the present embodiments which is referred to herein as NC₁₂K(NC₈K)₇NH₂, corresponds to a polymer having the general Formula I described hereinabove, wherein: X is a residue of a conjugate of an ω-amino-fatty acid having 12 carbon atoms (12-amino-lauric acid) and lysine; n is 6; A₁...A₆ are each a lysine residue; D₁...D₇ are all residues of an ω-amino-fatty acid having 8 carbon atoms (8-amino-caprylic acid); Z₁...Z₇ and W₀-W₇ are all peptide bonds; and Y is an amine. For clarity, the chemical structure of NC₁₂K(NC₈K)₇NH₂ is presented in Scheme 1 below:

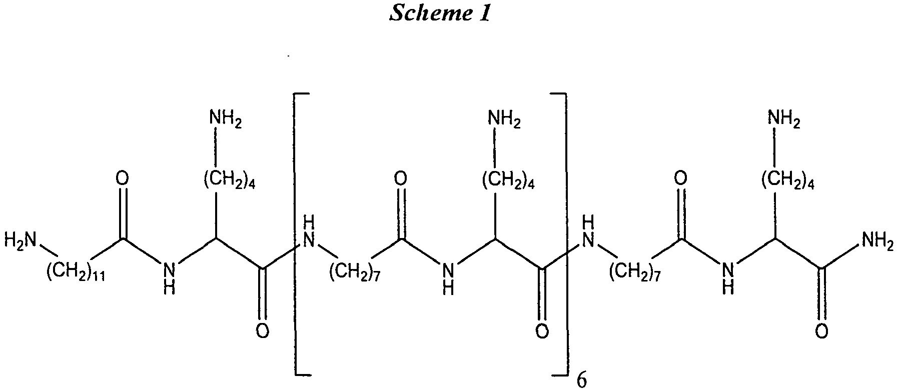

For another example, a polymer according to the present invention which is referred to herein as C₁₂K(ε)NC₁₂K(ε)NH₂, corresponds to a polymer having the general formula I described hereinabove, wherein: X is a residue of a conjugate of an ω-amino- fatty acid having 12 carbon atoms (12-amino-lauric acid) and lysine; n is 61 hence not denoted; A₁...A₆ A₂ are each a lysine residue, both conjugated via the epsilon amine hence denoted K(ε); D₁...D₇ are all is a residues of an ω-amino- fatty acid having 8 12 carbon atoms (812-amino-caprylic lauric acid); Z₁...Z₇ Z₂ and W₀-W₇₁ are all peptide bonds; and Y is an amine. For clarity, the chemical structure of C₁₂K(ε)NC₁₂K(ε)NH₂ is presented in Scheme 2 below:

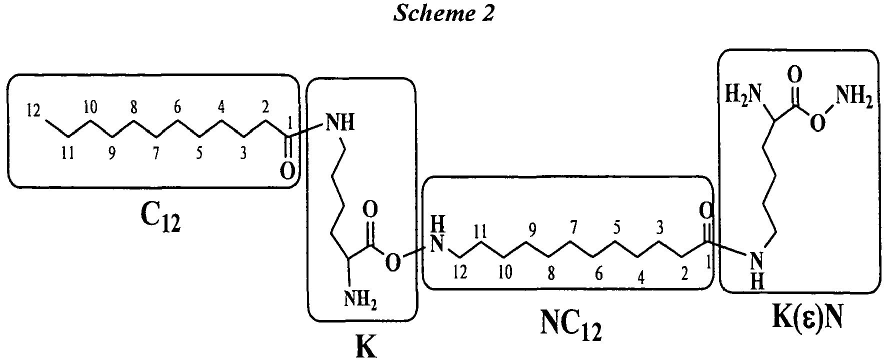

### Initial polymer library screen:

In order to extract the most active polymers out of a large library containing more than a hundred polymers, the library was initially screened against MCF-7 human breast cancer cells at a single concentration of 16 µM. The results obtained for the twenty most active polymers, as determined by this screen, are summarized in Figure 2 and Table 3 below.

Figure 2 presents a comparative bar graph showing the percent cell death as a result of exposure of the MCF-7 cells to 5 series, each series consisting of 4 exemplary polymers, differing in length and various chemical groups, such that the polymers in each series have a common core sequence having a certain number of repeating KNC₁₂K units (as defined above) and each polymer in each series has a certain chemical group selected from the group consisting of NC₁₂K, C₁₂K, C₈K and K or NC₁₂, C₁₂ and C₈ attached to the N-terminus thereof, demonstrating the anticancerous potential of the polymers as presented herein, and displaying the structure-activity relations and particularly the contribution of the length and net positive charge on the polymer on the cancerous-cell killing capacity thereof.

As can be deduced by structure-activity relationship (SAR) analysis of the results presented in Figure 2, the most potent polymers share some structural properties. These results suggest that activity is increased with length (number of subunits) and net positive charge, and to a lesser extent with increasing hydrophobicity of the polymer. A more detailed SAR analysis based on further studies follows below.

### Lethal and lytic concentration determination:

The polymers in each series were further tested for various cancer cell killing and hemotoxic activities, as described hereinabove. The obtained results are presented in Table 3 below, wherein:
"Charge" represents the overall molecular charge at physiological pH (column 3 in Table 3);
"Hydrophobicity (% ACN)" represents the percent of acetonitrile in an HPLC gradient mobile phase at which the polymer was eluted on a C₁₈ column using reverse-phase HPLC and which corresponds to the estimated hydrophobicity of the polymer (column 4 in Table 3);
"Cancer LC₅₀" represents the lethal concentration of each tested polymer in µM that induced death of 50 % of the specified cells after 24 hours incubation. (columns 5-8 in Table 3; values were extracted from dose-response curves and represent the mean ± standard deviation of two independent experiments each performed in duplicates; the values were rounded off to the nearest integer);
"Mouse prostate cancer TRAMP-C2" represents the lethal concentration in µM as measured against TRAMP-C2 cell line, measured as described hereinabove in the cell proliferation XTT assay (column 5 in Table 3);
"Human breast cancer MCF-7" represents the lethal concentration in µM as measured against MCF-7 cell line, measured as described hereinabove in the cell proliferation XTT assay (column 6 in Table 3);
"Human small-cell lung carcinoma N-417" represents the lethal concentration in µM as measured against N-417 cell line, measured as described hereinabove in the cell proliferation XTT assay (column 7 in Table 3);
"Rat primary cardiac fibroblast CF" represents the lethal concentration in µM as measured against CF cell line, measured as described hereinabove in the cell proliferation XTT assay (column 8 in Table 3);
"Hemolytic LC₅₀" represents the lytic concentration of each tested polymer in µM that induced lysis of 50 % of human red blood cells (RBC) after 3 hours incubation at 37 °C, measured as described hereinabove in the hemoglobin leakage assay (column 9 in Table 3; values were extracted from dose-response curves and represent the mean ± standard deviation of two independent experiments each performed in duplicates; the values were rounded-off to the nearest integer).

ND denotes "not determined".

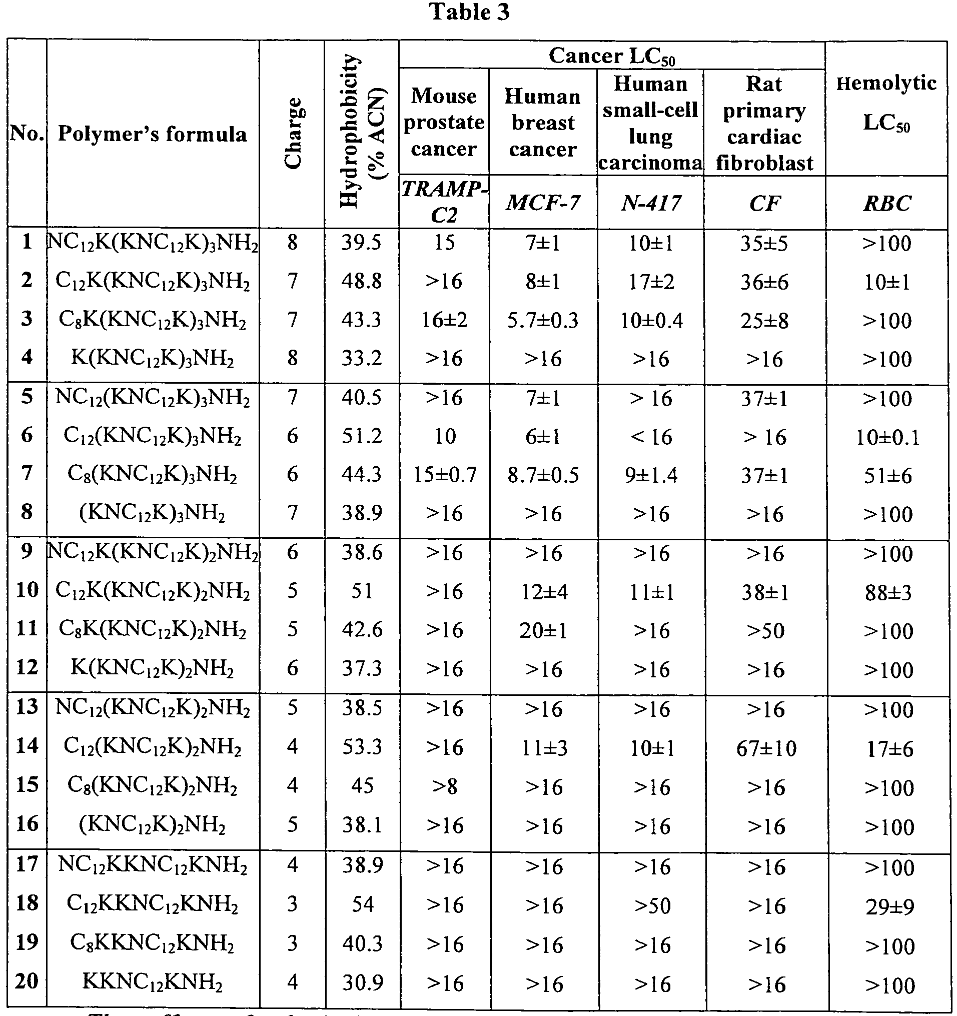

***The effect of physical parameters (charge and hydrophobicity)** on **anticancerous activity:***
Charge and hydrophobicity may be viewed as two conflicting physical characteristics of a molecule: charge facilitates dissolution of a compound in aqueous media by interacting with the polar water molecules, while hydrophobicity, which typically corresponds to the number and length of non-polar hydrocarbon moieties, hinders dissolution. Optimization of these physical characteristics is crucial in the development of drugs in general and anticancerous agents in particular, as these characteristics affect pharmaceutically important traits such as membrane permeability and transport in and across biological systems.

A serial increase in positive charge was achieved by preparing polymers with serial elongation of the chain with respect to the number of lysine residues. Serial increases in hydrophobicity was achieved by preparing polymers with serial rising of the number of fatty acid residues (as a representative hydrophobic moiety) and/or with serial rising of the number of carbon atoms in each fatty acid residue, for example, caprylic acid having 8-carbon long chain versus lauric acid having 12-carbon long chain. Serial increases in both positive charge and hydrophobicity were achieved by preparing polymers with serial rising of the number of lysine-amino fatty acid conjugates.

The library of polymers prepared to study the effect of serial increases in charge and hydrophobicity properties was assayed for its anticancerous activity against MCF-7 human breast cancer cells, as described hereinabove and against two additional cancerous cell lines: TRAMP-C2 cell line (results are presented in column 5 in Table 3 hereinabove) and N-417 cell line (results are presented in column 7 in Table 3 hereinabove).

As can be seen in Figure 2, increasing the hydrophobicity of the polymers by increasing the number of the carbon atoms in the fatty acid residue from 8 to 12, was found to affect the anticancerous activity of the polymers. Series of polymers in which the terminal hydrophobic moiety was a 8-amino-caprylic acid (see, entries 3, 7, 11, 15 and 19 in Table 3) was compared to a series in which the terminal hydrophobic moiety was a 12-amino-lauric acid (see, entries 2, 6, 10, 14 and 18 in Table 3). The results, presented in Figure 2, indicated that polymers in which the terminal hydrophobic moiety was an 8-amino-caprylic acid, generally exhibited equal or lower activity.

Evaluation of the effect of the hydrophobicity of the polymers in terms of the acetonitrile percentages of the HPLC mobile phase in which the polymers were eluted further demonstrates the correlation between this property and the anticancerous activity of the polymer. As can be seen in Figure 2 and Table 3, the polymers which have a higher number and longer hydrophobic chains displayed a more significant level of anticancerous activity.

As can further be seen in Figure 2 and Table 3, increasing the positive charge of the polymers by increasing the number of the lysine residues or adding an amino group to the end-subunit in the polymer was found to affect the anticancerous activity of the polymers only marginally.

Overall, these results indicate that anticancerous activity emerged when a polymer attained an optimal window of charge and hydrophobicity. These results also suggest that a parallel increase in hydrophobicity value might enhance potency. A minimal length of at least two repeating (KNC₁₂K) moieties and an overall "hydrophobicity" higher than 38 % acetonitrile emerge as prerequisite for potent cytotoxic/anticancerous activity. The presence of a hydrophobic (acyl) group at the N-terminus of the polymers appears to be crucial for enhancing cytotoxic activity. The presence of amino-acyl at the amino-terminus, yielding only a moderate increase in hydrophobicity due to the added positive charge, did not enhance the cytotoxic activity of relatively short polymers such as NC₁₂(KNC₁₂K)₂NH₂ (entry 13 in Table 3). However, longer polymer sequences such as present in NC₁₂(KNC₁₂K)₃NH₂ (entry 5 in Table 3) and NC₁₂K(KNC₁₂K)₃NH₂ (entry 1 in Table 3), exhibited remarkable cytotoxic effect on at least one of the tested cancer cell types.

In summary, is can be seen that anticancerous activity of the tested polymers correlates to long polymer sequences that are characterized by moderate hydrophobicity and high positive charge such as +7 or +8.

Other series of polymers, each series having either a certain overall charge and a certain hydrophobicity, wherein each polymer has a certain repeat of an NC₁₂K, NC₈K, NC₆K or NC₄K unit, or pairs of linear and cyclic polymers, or a series of polymers having a variety of positively charged amino acids, were also prepared and tested for their activity as anticancerous agents, and are presented in Table 4 below, whereas the column headers are:
"Q" represents the overall molecular charge at physiological pH (column 3 in Table 4);
"ACN (%)" represents the percent of acetonitrile in the HPLC-RP gradient mobile phase at which the polymer was eluted and which corresponds to the estimated hydrophobicity of the polymer (column 4 in Table 4);
"C6" represents the lethal concentration in µM as measured against C6 glioma cell line, which is a widely used cell line in neurobiological research (compared to primary neural cells, the C6 cell line offers some major advantages like easy accessibility and culture as well as a good availability of high cell numbers), (column 5 in Table 4);
"MCF-7" represents the lethal concentration in µM as measured against human breast adenocarcinoma cells MCF-7 cell line, measured as described hereinabove in the cell proliferation XTT assay (column 6 in Table 4);
"CEM" represents the lethal concentration in µM as measured against human CEM T-cell line, which is MDR induced as described in Oerlemans, R. et al., Arthritis & Rheumatism, 2006, 54(2), pp. 557-568 (column 7 in Table 4);
"N-417" represents the lethal concentration in µM as measured against human small-cell lung carcinoma N-417 cell line, measured as described hereinabove in the cell proliferation XTT assay (column 8 in Table 4);
"TRAMP-C2" represents the lethal concentration in µM as measured against transgenic adenocarcinoma mouse prostate cancer TRAMP-C2 cell line, measured as described hereinabove in the cell proliferation XTT assay (column 9 in Table 4);
"CF" represents the lethal concentration in µM as measured against primary non-cancerous rat cardiac fibroblasts (CF) cell line, measured as described hereinabove in the cell proliferation XTT assay (column 10 in Table 4); and
"LC₅₀ RBC" represents the hemolytic concentration of each tested polymer in µM that induced lysis of 50 % of human red blood cells (RBC) after 3 hours incubation at 37 °C, measured as described hereinabove in the hemoglobin leakage assay (column 11 in Table 4;
and whereas the values were extracted from dose-response curves and represent the mean ± standard deviation of two independent experiments each performed in duplicates; the values were rounded-off to the nearest integer; and ND denotes "not determined".

**Table 4**

| **No.** | **Polymer** | **Q** | **ACN (%)** | **C6** | **MCF-7** | **CEM** | **N-417** | **TRAMP -C2** | **CF** | **LC50 RBC** |
|---|---|---|---|---|---|---|---|---|---|---|
| **1** | C₈KNC₁₂KNH₂ | 2 | 45 | ND | >16 | >16 | >16 | >16 | ND | ND |
| **2** | C₁₂KNC₁₂KNH₂ | 2 | 54.4 | >16 | ND | ND | ND | ND | >16 | 45±12 |
| **3** | C₁₂K(ε)NC₁₂K(ε)NH₂ | 2 | 54.4 | >16 | >16 | >16 | >16 | >16 | ND | ND |
| **4** | C₁₆KNC₁₂KNH₂ | 2 | 69 | ND | 16 | >16 | >16 | >16 | ND | ND |
| **5** | C₁₂K(NC₁₂K)₂NH₂ | 3 | 52.9 | >16 | ND | ND | ND | ND | >16 | ND |
| **6** | C₁₂K(NC₁₂K)₃NH₂ | 4 | 53.5 | >16 | ND | ND | ND | ND | >16 | ND |
| **7** | C₁₂K(NC₁₂K)₄NH₂ | 5 | 53.4 | >16 | 7.3±0.3 | >16 | >16 | ND | 37±2.12 | ND |
| **8** | KNC₁₂KNH₂ | 3 | 32 | >16 | ND | ND | ND | ND | >16 | >100 |
| **9** | K(NC₁₂K)₂NH₂ | 4 | 40 | >16 | ND | ND | ND | ND | >16 | >100 |
| **10** | K(NC₁₂K)₃NH₂ | 5 | 44 | >16 | ND | ND | ND | ND | >16 | >100 |
| **11** | Cyclic-K(NC₁₂K)₃ | 4 | 44.2 | ND | >16 | >16 | >16 | ND | ND | ND |
| **12** | K(NC₁₂K)₄NH₂ | 6 | 46 | >16 | 11±2.8 | >16 | 14.5±0.7 | ND | 50 | 6.5±3.5 |
| **13** | K(NC₁₂K)₅NH₂ | 7 | 47 | >16 | ND | ND | ND | ND | >16 | ND |
| **14** | Cyclic-K(NC₁₂K)₅ | 6 | 46.6 | >16 | 10.5±0.5 | >16 | >16 | >16 | ND | 3.4±0.3 |
| **15** | K(NC₁₂K)₆NH₂ | 8 | 48 | >16 | ND | ND | ND | ND | >16 | ND |
| **16** | K(NC₁₂K)₇NH₂ | 9 | 50 | >16 | ND | ND | ND | ND | >16 | ND |
| **17** | (NC₁₂K)₂NH₂ | 3 | 38.8 | >16 | ND | ND | ND | ND | >16 | >100 |
| **18** | Cyclic-(NC₁₂K)₂ | 2 | 49.7 | >16 | >16 | ND | ND | >16 | ND | ND |
| **19** | (NC₁₂K)₃NH₂ | 4 | 44.3 | >16 | ND | >16 | >16 | ND | >16 | >100 |
| **20** | Cyclic-(NC₁₂K)₃ | 3 | 47.0 | >16 | >16 | >16 | >16 | >16 | ND | ND |
| **21** | (NC₁₂K)₄NH₂ | 5 | 46.8 | ND | ND | ND | ND | ND | ND | 4±1.4 |
| **22** | Cyclic-(NC₁₂K)₄ | 4 | 47.4 | >16 | >16 | >16 | >16 | >16 | ND | ND |
| **23** | (NC₁₂K)₅NH₂ | 6 | 47.8 | >16 | ND | ND | ND | ND | >16 | ND |
| **24** | Cyclic-(NC₁₂K)₅ | 5 | 47.7 | >16 | >16 | >16 | >16 | >16 | ND | ND |
| **25** | (NC₁₂K)₆NH₂ | 7 | 49 | >16 | ND | ND | ND | ND | >16 | ND |
| **26** | (NC₁₂K)₇NH₂ | 8 | 50 | >16 | ND | ND | ND | ND | >16 | ND |
| **27** | (NC₁₂K)₈NH₂ | 9 | 51 | >16 | ND | ND | ND | ND | >16 | ND |
| **28** | (KNC₁₂K)₂NH₂ | 5 | 38.1 | ND | >16 | ND | >16 | >16 | >16 | >100 |
| **29** | Cyclic-(KNC₁₂K)₂ | 4 | 40.7 | >16 | >16 | ND | ND | >16 | ND | >100 |
| **30** | (KNC₁₂K)₃NH₂ | 7 | 38.9 | ND | >16 | ND | >16 | >16 | >16 | >100 |
| **31** | Cyclic-(KNC₁₂K)₃ | 6 | 41.7 | >16 | >16 | ND | ND | >16 | ND | >100 |
| **32** | C₁₆KKNC₁₂KNH₂ | 3 | >16 | >16 | ND | ND | >16 | ND | ND | ND |
| **33** | C₁₆(1)KKNC₁₂KNH₂ | 3 | 57 | ND | 9±1 | ND | ND | ND | ND | ND |
| **34** | C₁₈(1)KKNC₁₂KNH₂ | 3 | 63 | >16 | >16 | ND | ND | >16 | ND | ND |
| **35** | Cyclic-NC₁₂KKNC₁₂K | 3 | 42.7 | >16 | >16 | ND | ND | >16 | ND | >100 |
| **36** | NC₁₂K(KNC₁₂K)₂NH₂ | 6 | 38.6 | ND | ND | ND | ND | ND | ND | ND |
| **37** | Cyclic-NC₁₂K(KNC₁₂K)₂ | 5 | 43.1 | >16 | >16 | ND | ND | >16 | ND | >100 |
| **38** | C₁₂(KNC₈K) NH₂ | 2 | ND | >16 | >16 | ND | >16 | >16 | <16 | ND |
| **39** | C₁₂(KNC₈K)₂ NH₂ | 4 | ND | >16 | >16 | ND | >16 | >16 | <16 | ND |
| **40** | C₁₂(KNC₈K)₃ NH₂ | 6 | ND | >16 | >16 | ND | >16 | >16 | <16 | ND |
| **41** | C₁₂(KNC₈K)₄ NH₂ | 8 | ND | >16 | >16 | ND | >16 | >16 | <16 | ND |
| **42** | KNC₁₂NC₁₂KNH₂ | 2 | 43 | >16 | >16 | ND | >16 | >16 | ND | ND |
| **43** | C₁₂KNC₁₂NC₁₂KNH₂ | 2 | 43 | >16 | >16 | ND | >16 | >16 | ND | ND |
| **44** | C₁₂KKNC₁₂NC₁₂KNH₂ | 3 | 57 | >16 | 12.5±1 | >16 | >16 | 10.5±0.5 | ND | ND |
| **45** | C₁₂KKNC₄KNH₂ | 3 | 44 | >16 | >16 | ND | >16 | >16 | ND | ND |
| **46** | C₁₂KKNC₄(α)KNH₂ | 3 | 46 | >16 | >16 | ND | >16 | >16 | ND | ND |
| **47** | C₆NC₆KNC₁₂KNH₂ | 2 | 42 | >16 | >16 | ND | >16 | >16 | ND | ND |
| **48** | C₁₂KNC₆NC₆KNH₂ | 2 | 50 | >16 | >16 | ND | >16 | >16 | ND | ND |
| **49** | C₆NC₆KNC₆NC₆KNH₂ | 2 | 33 | >16 | >16 | ND | >16 | >16 | ND | ND |
| **50** | C₆NC₆KKNC₆NC₆KNH₂ | 3 | 23 | >16 | >16 | ND | >16 | >16 | ND | ND |
| **51** | C₆NC₆KKNC₁₂KNH₂ | 3 | 38 | >16 | >16 | ND | >16 | >16 | ND | ND |
| **52** | C₁₂KKNC₆NC₆KNH₂ | 3 | 46 | >16 | >16 | ND | >16 | >16 | ND | ND |
| **53** | C₁₂OrnNC₁₂OrnNH₂ | 2 | 53.8 | ND | ND | ND | ND | ND | ND | 24±6 |
| **54** | C₁₂ArgNC12ArgNH₂ | 2 | 57.1 | ND | ND | ND | ND | ND | ND | 9.5±1 |
| **55** | C₁₂KNC₁₂K | 1 | 56.9 | ND | ND | ND | ND | ND | >16 | >100 |
| **56** | C₁₂K(NC₁₂K)₂ | 2 | 56.4 | ND | ND | ND | ND | ND | >16 | ND |
| **57** | C₁₂K(NC₁₂K)₃ | 3 | 54.6 | ND | ND | ND | ND | ND | >16 | ND |
| **58** | KNC₁₂K | 2 | 33.2 | ND | ND | ND | ND | ND | >16 | >100 |
| **59** | K(NC₁₂K)₂ | 3 | 36.4 | ND | ND | ND | ND | ND | >16 | >100 |
| **60** | K(NC₁₂K)₃ | 4 | 42.8 | ND | ND | ND | ND | ND | >16 | >100 |
| **61** | (NC₁₂K)₂ | 2 | 38.7 | ND | ND | ND | ND | ND | >16 | >100 |
| **62** | (NC₁₂K)₃ | 3 | 43.8 | ND | ND | ND | ND | ND | >16 | >100 |
| **63** | (NC₁₂K)₄ | 4 | 45.8 | ND | ND | ND | ND | ND | >16 | ND |
| **64** | FmocK(NC₁₂K)₂ | 2 | 41 | ND | ND | ND | ND | ND | ND | ND |
| **65** | Cyclic-KNC₁₂KKKNC₁₂K | 5 | 38 | ND | ND | ND | ND | ND | ND | ND |
| **66** | Cyclic-KNC₁₂KKKNC₁₂KK | 6 | 35 | ND | ND | ND | ND | ND | ND | ND |
| **67** | C₁₂KNC₆NC₆KNH₂ | 2 | 50 | ND | ND | ND | ND | ND | ND | ND |
| **68** | C₆NC₆KNC₁₂KNH₂ | 2 | 42 | ND | ND | ND | ND | ND | ND | ND |
| **69** | C₆NC₆KNC₆NC₆KNH₂ | 2 | 33 | ND | ND | ND | ND | ND | ND | ND |
| **70** | C₁₂KKNC₆NC₆KNH₂ | 3 | 46 | ND | ND | ND | ND | ND | ND | ND |
| **71** | C₆NC₆KKNC₁₂KNH₂ | 3 | 38 | ND | ND | ND | ND | ND | ND | ND |
| **72** | C₆NC₆KK NC₆NC₆K NH₂ | 3 | 23 | ND | ND | ND | ND | ND | ND | ND |
| **73** | KNC₁₂ NC₁₂KNH₂ | 2 | 43 | ND | ND | ND | ND | ND | ND | ND |
| **74** | C₁₂KNC₁₂ NC₁₂KNH₂ | 2 | 63 | ND | ND | ND | ND | ND | ND | ND |
| **75** | C₁₂KKNC₁₂NC₁₂KNH₂ | 3 | 57 | ND | ND | ND | ND | ND | ND | ND |
| **76** | C₁₂(1)KNC₁₂KNH₂ | 2 | 54 | ND | ND | ND | ND | ND | ND | ND |
| **77** | C₁₄(1)KNC₁₂KNH₂ | 2 | 58 | ND | ND | ND | ND | ND | ND | ND |
| **78** | C₁₆(0)KNC₁₂KNH₂ | 2 | 69 | ND | ND | ND | ND | ND | ND | ND |
| **79** | C₁₆(1)KNC₁₂KNH₂ | 2 | 63 | ND | ND | ND | ND | ND | ND | ND |
| **80** | C₁₂(1)KKNC₁₂KNH₂ | 3 | 50 | ND | ND | ND | ND | ND | ND | ND |
| **81** | C₁₄(1)KKNC₁₂KNH₂ | 3 | 52 | ND | ND | ND | ND | ND | ND | ND |
| **82** | C₁₆(0)KKNC₁₂KNH₂ | 3 | 62 | ND | ND | ND | ND | ND | ND | ND |
| **83** | C₁₆(1)KKNC₁₂KNH₂ | 3 | 57 | ND | ND | ND | ND | ND | ND | ND |
| **84** | C₁₈(0)KKNC₁₂KNH₂ | 3 | 63 | ND | ND | ND | ND | ND | ND | ND |
| **85** | C₁₈(1)KKNC₁₂KNH₂ | 3 | 60 | ND | ND | ND | ND | ND | ND | ND |
| **86** | C₈(2)KKNC₁₂KNH₂ | 3 | 57 | ND | ND | ND | ND | ND | ND | ND |
| **87** | C₁₂KKN((α)C₄KNH₂ | 3 | 46 | ND | ND | ND | ND | ND | ND | ND |
| **88** | C₁₂K(NC₈K)₇-Glucose | 8 | 45.7 | ND | ND | ND | ND | ND | ND | ND |
| **89** | NBD-NC₁₂K(NC₈K)₇ NH₂ | 8 | 45 | ND | ND | ND | ND | ND | ND | ND |
| **90** | C₁₂(KNC₈K)₂NH₂ | 4 | 48.3 | ND | ND | ND | ND | ND | ND | ND |
| **91** | C₁₂(KNC₈K)₃NH₂ | 6 | 46.8 | ND | ND | ND | ND | ND | ND | ND |
| **92** | C₁₂(KNC₈K)₄NH₂ | 8 | 45.9 | ND | ND | ND | ND | ND | ND | ND |
| **i** | Mitomycin C | - | ND | ND | 1.4±0.9 | ND | ND | ND | ND | ND |
| **ii** | Doxorubicin | - | ND | ND | 3.3±2 | ND | ND | 0.5±0.5 | ND | ND |

As can be seen in Table 4, cyclic polymers wherein a covalent bond connects between the N-terminal amine and the C-terminal carboxyl of the polymer (see, entries 11, 14, 18, 20, 22, 24, 29, 31, 35 and 37 in Table 4) were prepared in order to be compared to their linear counterparts having an amide instead of a C-terminal carboxyl (see, respective entries 10, 13, 17, 19, 21, 23, 28, 30, 34 and 36 in Table 4).

As can further be seen in Table 4, other polymers having unique structural characteristics, such as C₁₂K(ε)NC₁₂K(ε)NH₂ (see, entry 3 in Table 4), wherein one of the lysine residues is attached via the ε-amine instead of α-amine; C₁₂KKNC₄(α)KNH₂ (see, entry 46 in Table 4), wherein an α-aminobutyric acid residue is used as a hydrophobic moiety instead of γ-aminobutyric acid residue; C₁₆(1)KKNC₁₂KNH₂ (see, entry 33 in Table 4), wherein palmitoleic acid residue (unsaturated fatty acid) is used as a hydrophobic moiety at the N-terminus; C₁₈(1)KKNC₁₂KNH₂ (see, entry 33 in Table 4), wherein oleic acid residue is used as a hydrophobic moiety at the N-terminus; polymers using ornithine and arginine as positively charged amino acid residues (see, entries 53 and 54 respectively in Table 4); and a polymer having an amine protecting group attached to its N-terminus (see, entry 64 in Table 4).

Other examples of polymers having unique structural characteristics include C₁₂(1)KKNC₁₂KNH₂ wherein the hydrophobic moiety of lauroleic acid is attached at the N-terminus of the polymer, C₁₄(1)KKNC₁₂KNH₂ wherein the hydrophobic moiety of myristoleic acid is attached at the N-terminus of the polymer, C₁₆(0)KKNC₁₂KNH₂ wherein the hydrophobic moiety of palmitic acid is attached at the N-terminus of the polymer, C₁₆(1)KKNC₁₂KNH₂ wherein the hydrophobic moiety of palmitoleic acid is attached at the N-terminus of the polymer, C₁₈(1)KKNC₁₂KNH₂ wherein the hydrophobic moiety of oleic acid is attached at the N-terminus of the polymer, C₁₈(2)KKNC₁₂KNH₂ wherein the hydrophobic moiety of linoleic acid is attached at the N-terminus of the polymer, and C₁₈(3)KKNC₁₂KNH₂ wherein the hydrophobic moiety of linolenic acid is attached at the N-terminus of the polymer.

Exemplary polymers, having an additional active agent attached thereto include C₁₂K(NC₈K)₇-Glucose (see, entry 88 in Table 4) having D(+)-Glucosamine hydrochloride attached at the C-terminus of the polymer, and NBD-NC₁₂K(NC₈K)₇ (see, entry 89 in Table 4) having the labeling agent nitrobenzoxadiazole fluorophore attached to the N-terminus of the polymer.

Entries i and ii in Table 4 present the experimental values obtained under similar conditions for two commonly used chemotherapeutic agents.

### Selective and specific anticancerous activity:

To evaluate the specificity of the anticancerous polymers presented herein, namely a selectivity towards cancer cells and inactivity towards non-cancerous cells, and further a specificity towards a particular cell type, the polymers that were found most active against human MCF-7 cells were further tested, at a fixed concentration of 16 µM, against human N-417 cancerous cell line and against rat cardiac fibroblast (CF) non-cancerous cells.

Figure 3 presents a comparative bar graph showing the percent cell death as a result of exposure of rat cardiac fibroblast, human MCF-7 cells and human N-417 cells to 3 series of exemplary polymers, each series being of 3 polymers differing in length and various chemical groups.

As can be seen in Figure 3, all polymers exhibited very low activity against non-cancerous CF cells, indicating that these polymers can serve as promising anticancerous drugs with low side-effects and low risk to healthy tissues. As can further be seen in Figure 3, the various polymers did not exhibit identical activity against both types of cancers, indicating that the particular characteristics of the polymers, namely length, charge and hydrophobicity, can be used to design a more highly specific anticancerous agent. Thus, while non-acylated polymers, e.g., polymers having a free amine at one of the ends of the polymer, displayed a mild yet measurable activity and selectivity, the addition of a lauryl moiety at the end of the polymer, thereby turning it to an acylated polymer, has invariably increased its cytotoxic activity. Furthermore, substitution of the lauryl moiety by its amine-derivative had a differential effect that contributed to specific activity of relatively long, such as in the case of NC₁₂(KNC₁₂K)₃NH₂ (see, entry 5 in Table 3) and NC₁₂K(KNC₁₂K)₃NH₂ (see, entry 1 in Table 3).

Selectivity of the active polymers was further examined by determination of the individual LC₅₀ value.Figures 4a-b present a comparative plot of the dose-dependent activity of two representative anticancerous polymers, C₈K(KNC₁₂K)₃NH₂ (see, entry 3 in Table 3, Figure 4a) and C₁₂(KNC₁₂K)₂NH₂ (see, entry 14 in Table 3, Figure 4b).

As can be seen in Figures 4a-b, the LC₅₀ values which were calculated from these plots were 5.5 µM for C₈K(KNC₁₂K)₃NH₂ and 11 µM C₁₂(KNC₁₂K)₂NH₂ against MCF-7 cells (white rectangles), and 25 µM for C₈K(KNC₁₂K)₃NH₂ and 67 µM for C₁₂(KNC₁₂K)₂NH₂ against non-cancerous CF cells (gray triangles). These results clearly demonstrate the selective activity exhibited by the anticancerous polymers presented herein against cancerous cells versus non-cancerous cells.

As presented herein, exemplary polymers also exhibited selective anticancerous activity, showing a selectivity index (LC₅₀ ratio) of 4-fold or higher. Similarly, hemolytic concentrations of various anticancerous polymers, such as NC₁₂(KNC₁₂K)₃NH₂ (see, entry 5 in Table 3) and NC₁₂K(KNC₁₂K)₃NH₂ (see, entry 1 in Table 3) were often more than one order of magnitude higher than the corresponding cytotoxic concentrations these polymers exhibited against cancer cells.

### Kinetic cytotoxicity studies:

The kinetic rate of the cytolytic activity of three representative polymers, C₈K(KNC₁₂K)₃NH₂ (see, entry 3 in Table 3), C₁₂(KNC₁₂K)₃NH₂ (see, entry 6 in Table 3) and C₁₂(KNC₁₂K)₂NH₂ (see, entry 14 in Table 3), was determined as described in the methods section above against MCF-7, TRAMP-C2 and CF cells, at an 8-fold concentration of their respective LC₅₀ value, so as to assure 100 % cell death, and compared to that of the currently used chemotherapeutic drug mitomycin C (MytC) at the same concentration.

Figure 5 presents comparative plots demonstrating the kinetic cytotoxic activity of C₈K(KNC₁₂K)₃NH₂ (marked in triangles) and C₁₂(KNC₁₂K)₂NH₂ (marked in rectangles), two exemplary anticancerous polymers according to the present invention, MCF-7 cells, as compared with kinetic cytotoxic activity of mitomycin C (marked in diamonds) as determined at a concentration corresponding to eight multiples of their respective LC₅₀ value.

As can be seen in Figure 5, the polymers C₈K(KNC₁₂K)₃NH₂ and C₁₂(KNC₁₂K)₂NH₂ exhibited rapid kinetics, killing the entire MCF-7 cell population within 20 minutes and 120 minutes, respectively. In sharp contrast Mytomicin C displayed markedly slower kinetics, inducing only 50 % lysis after 3 hours incubation under the same conditions as for the polymers.

Figures 6a-b present the cytotoxic aptitude of C₁₂(KNC₁₂K)₃NH₂ (see, entry 6 in Table 3), an exemplary anticancerous polymer, showing the polymer's selectivity towards killing cancerous cells, exhibiting an LC₅₀ value of 11 µM towards TRAMP-C2 cells at (see, Figure 6a, marked by diamonds), as compared to a much higher LC₅₀ value of 38 µM towards benign CF cells (see, Figure 6a, marked by triangles) measured in dose-dependent comparative plot. The rapid rate of cytotoxicity, as measured in a time-dependent activity plot is presented in Figure 6b.

### Cellular localization:

In order to investigate the potential targets of the anticancerous polymers presented herein, the cellular localization of the polymers was determined by chemical fluorescent labeling.

As mitochondria have been reported as potential targets of AMPs, an exemplary polymer, NC₁₂(KNC₁₂K)₃NH₂ (see, entry 5 in Table 3) was selectively labeled with a fluorescent marker (NBD-F) as described in the methods section hereinabove. The NBD label did not significantly alter the polymer's cytolytic properties (about 20 % variation in activity). A sub-lytic concentration (20 µM) of the NBD-labeled polymer was incubated for 30 minutes with TRAMP-C2 cells, and the culture was exposed to the MitoTracker Red.

Figure 7 presents a photograph taken on a confocal fluorescence microscope (magnification: 1 cm = 5 microns) of two cells in a TRAMP-C2 cell culture treated for 30 minutes with 20 µM of NC₁₂(KNC₁₂K)₃NH₂, an exemplary polymer of the present invention, labeled with NBD, and further incubated with a mitochondrial specific marker MitoTracker Red, showing the cellular localization of the polymer inside the cell and distribution throughout the cytoplasm.

As can be seen in Figure 7, the NBD-labeled polymer was distributed quasi-evenly throughout the cytoplasm of TRAMP-C2 cells, while no evidence could be obtained for localization either at the cell-membrane or the nucleus. It is therefore assumed that the polymers induce their anticancerous effect through interaction with soluble cytoplamic component(s) such as the mitochondria.

Such target components may include a plethora of negatively charged molecules, proteins, nucleic acids and the likes, including those participating in signal transduction pathways downstream to oncogen or tumor suppressor genes products [Winder, D. et al, Biochem. Biophys. Res. Commun., 1998, 242, 608-614].

### Tumor induction and treatment:

Adenocarcinoma tumors were induced in animal models by subcutaneous injection of TRAMP-C2 cells (2.5 X 10⁶ TRAMP-C2 cells suspended in 0.1 ml of cell medium comprised of DMEM supplemented with 2 mM L-glutamine, 100 mg/ml penicillin-streptomycin, 5 µg/ml insulin, and 10⁻⁸ M di-hydrotestosterone) into the flank area of 4-6 week-old syngeneic (isogeneic) C57BL/6 mice.

Tumor evolution was monitored by measuring tumor sizes with a caliper twice a week over a period of 28 days. At the 28^{th} day the mice were sacrificed, and the tumors were removed and weighed. The animal experimentation was reviewed and approved by the Animal Care and Use Committee.

The *in vivo* effect of NC₁₂K(KNC₁₂K)₃, an exemplary anticancerous polymer according to the present embodiments, was assessed in two distinct experiments. In the initial experiment treatment started at the 7^{th} day when tumor volume reached 40-50 mm³, and the results are presented in Figures 8A-E.

Figures 8A and B show growth curves for each mouse when treated by a daily injection of a blank control PBS (n=14) (Figure 8A) or 5 mg/kg of NC₁₂K(KNC₁₂K)₃ (n=11) (Figure 8B). Treatment started when tumor volume reached 40-50 mm³ as indicated by an arrow in both Figures 8A and B.

Figure 8C shows a graph of the measured weights of tumor extracted at the 28^{th} day post inoculation from tumor-induced mice treated with PBS (control) or NC₁₂K(KNC₁₂K)₃, at the specified doses, namely 2.5 mg/kg and 5 mg/kg.

Figures 8D and E show representative tumor bearing mice which were treated with NC₁₂K(KNC₁₂K)₃ (Figure 8D), or treated with the control (Figure 8E).

As can be seen in Figures 8A and B, NC₁₂K(KNC₁₂K)₃inhibited significantly (P < 0.5) tumor progression in most of the 5 mg/kg treated mice, as indicated by the tumor volume measurements and confirmed post-mortem by tumor weights. As expected in dose response measurements, NC₁₂K(KNC₁₂K)₃ displayed a milder effect at 2.5 mg/kg.

The ability of the polymers presented herein to prevent tumor emergence through early treatment (one day post inoculation) was also studied. Under these conditions, NC₁₂K(KNC₁₂K)₃ did not prevent tumor emergence since all mice (treated and control) have eventually developed a measurable tumor. However, this study confirmed the polymer's ability to inhibit tumor growth in at least 5 out of 7 treated mice, as shown in Figure 9

Figures 9A-E present the effect of early treatment of C57BL/6 mice bearing TRAMP-C2 allograft with NC₁₂K(KNC₁₂K)₃. Treatment started 24 hours after inoculation, indicated by an arrow (Figures 9A and B).

Figures 9A and B show growth curves for each mouse when treated by a daily injection with PBS (n=7) (Figure 9A) or 5 mg/kg of NC₁₂K(KNC₁₂K)₃ (n=7) (Figure 9B).

Figures 9C and D show two bar graphs presenting the weight of tumors extracted from mice treated with PBS (Figure 9C) or NC₁₂K(KNC₁₂K)₃ (Figure 9D) on the 28^{th} day post inoculation, and Figure 9E shows photographs of the extracted and measured tumors.

As can be seen in Figure 9, tumor progression was significantly inhibited as indicated by the tumor volume measurements (Figure 9B) and as confirmed post-mortem by tumor weights (Figure 9D). As expected in dose response measurements, NC₁₂K(KNC₁₂K)₃ displayed a milder effect on 1/7 and did not affect at all 1/7 of the treated mice.

### Activity in drug-resistant versus drug-sensitive cell lines:

The cytotoxic activity of the polymers was studied in two related cancer cell lines, one which is known to be drug-sensitive (native cell line) and the other known to be drug-resistant, in order to estimate the effectiveness of the anticancerous polymers presented herein in MDR cancer cases.

Human ovarian carcinoma drug-sensitive cell line 2008 and its stable MRP1 transfectant and drug resistant variant cell line (i.e., canalicular multispecific organic anion transporter) were cultured in RPMI 1640, supplemented with 10 % (v/v) heat-inactivated fetal calf serum, 2 mM glutamine, and 100 mg/ml penicillin-streptomycin.

The cells were plated in 100 µL of medium at an initial density of 5 X 10³ cells per well of 96-well plate and incubated at humidified 5 % CO₂ atmosphere at 37 °C for 24 hours. One day after cell plating, the culture medium was replaced with 100 µL fresh medium containing NC₁₂K(KNC₁₂K)₃, an exemplary polymer according to preferred embodiments, in two-fold serial dilutions.

The cytotoxic effect was established by measuring optical density of cells at 450 nm, and the results are presented in Figure 10 and Figure 11.

Figure 10 presents a comparative plot of the dose dependent cytotoxic effect of NC₁₂K(KNC₁₂K)₃ against ovarian carcinoma 2008 drug-sensitive wild type cells (shown in black rectangles) and against drug-resistant 2008/MRP1 cells (in red circles) after overnight incubations.

As can be seen in Figure 10, after 1 day exposure, NC₁₂K(KNC₁₂K)₃ displayed similar potency against the drug-sensitive human ovarian carcinoma 2008 cells as compared to its potency against the drug-resistant 2008/MRP1 cells (LC₅₀ = about 15 µM).

Figures 11A and B present two comparative plots of the time dependent cytotoxic effect (time-kill curves) of NC₁₂K(KNC₁₂K)₃ against the drug-resistant 2008/MRP1 cells (Figure 11A) and the drug-sensitive human ovarian carcinoma 2008 cells (Figure 11B), showing that the cytotoxic effect is extremely rapid in terms of minutes. Each data point represents the mean of two experiments performed in duplicate cultures for each drug concentration, and the vertical bars represent one SD.

### Chemosensitization activity:

In order to establish the chemosensitization effect of the polymers presented herein, the anticancerous activity of a known anticancerous drug, doxorubicin, was estimated in the absence and presence of exemplary polymers according to the present embodiments, as measured in drug-resistant and drug-sensitive cell lines. Doxorubicin, also known as adriamycin or hydroxyldaunorubicin, is a DNA-binding drug which is widely used in chemotherapy.

Human ovarian carcinoma drug-sensitive 2008 cells and drug-resistant MRP1 cells were plated in 100 µL of medium at an initial density of 5 X 10³ cells per well of 96-well plate and incubated at humidified 5 % CO₂ atmosphere at 37 °C for 24 hours. One day after cell plating, the culture medium was replaced with 100 µL of fresh medium containing doxorubicin in ten-fold serial dilutions, or fresh medium containing NC₁₂K(KNC₁₂K)₃, an exemplary polymer according to preferred embodiments, in two-fold serial dilutions, or a combination of both doxorubicin and NC₁₂K(KNC₁₂K)₃.

In co-incubation experiments cells were exposed to a similar solution containing both drugs simultaneously in dilutions as specified above. Following 72 hours re-incubation, 50 µl XTT reaction solution were added for 2-4 hours additional incubation before measuring optical density at 450 nm. Statistical data was obtained from at least two independent experiments performed in duplicates, and the results are presented in Figure 12.

Figure 12 presents a comparative plot of the dose dependent cytotoxic effect of doxorubicin against drug-sensitive ovarian carcinoma 2008 cells (black triangles), doxorubicin against drug-resistant 2008/MRP1 cells (white triangles), NC₁₂K(KNC₁₂K)₃ against drug-resistant 2008/MRP1 cells (white triangles), and the cytotoxic effect of the combination of doxorubicin (varying dose) and NC₁₂K(KNC₁₂K)₃ (constant 4 µM concentration) against drug-resistant 2008/MRP1 cells (white rectangles), all after 3-day cultures. Each data point represents the mean of two experiments performed in duplicate cultures for each drug concentration, and the vertical bars represent one SD.

As can be seen in Figure 12, doxorubicin displayed potent activity against the drug-sensitive human ovarian carcinoma 2008 cells (LC₅₀ = 35 nM) and a 12.8-fold reduced potency against the drug-resistant 2008/MRP 1 cells after the long incubation periods of 3-days.

Under similar conditions the dose-dependent cytotoxic effect of NC₁₂K(KNC₁₂K)₃ had a steeper profile, displaying a more moderate activity as compared to doxorubicin (LC₅₀= 4 µM). However, in presence of NC₁₂K(KNC₁₂K)₃ at 4 µM, doxorubicin displayed an enhanced potency against the drug-resistant cells, resembling its effect over the wild type cells.

### Cytotoxic effect on drug-resistant cells (XTT assay):

The ability of an exemplary polymer according to the present embodiments, NC₁₂K(KNC₁₂K)₃NH₂ (entry 1 in table 3), to induce cytotoxic effect on drug-resistant cells overexpressing one of seven ATP-binding cassette (ABC) transporters, was tested using the aforementioned XTT assay on AA8 CHO parental cell-line and its transfectant EMTR1 overexpressing PGP, A549 non small cell lung cancer cell-line and its transfectant K1.5 overexpressing BCRP, 2008 cell-line and its 3 transductants overexpressing MRP1, MRP2 and MRP3, and parental ovarian carcinoma and overexpressing cells of the specified transporters, HEK cell-line and its transfectants MRP4 and MRP5 respectively. Briefly, these human parental and drug resistant cancer cell lines were grown in RPMI 1640 medium (Invitrogen™-GIBCO® Carlsbad, CA) supplemented with 10 % heat inactivated fetal calf serum, 2 mM L-glutamine, 100 µ/ml penicillin-streptomycin (Biological Industries, Beth-Haemek, Israel).

Figures 13A-D present comparative plots, showing the dose dependent cytotoxic effect of NC₁₂K(KNC₁₂K)₃NH₂, an exemplary polymer according to the present embodiments, as measured using the XTT assay on resistant and sensitive cells, namely against AA8 and EMTR1 cell-lines (marked in black rectangles and white circles respectively in Figure 13A); A549 and K1.5 cell-lines (marked in black rectangles and white circles respectively in Figure 13B); 2008, MRP1, MRP2 and MRP3 cell-lines (marked in black rectangles, white diamonds, white circles and white triangles respectively in Figure 13C); and against HEK, MRP4 and MRP5 cell-lines (marked in black rectangles, black circles and white diamonds in Figure 13D).

As can be seen in Figures 13A-D, the anticancerous polymer induced comparable LC₅₀ values, range of 15-18µM, of parental and resistant cells. As previously described for representative 2008 and MRP1 cells, the time-kill curves were also comparably rapid and dose dependant, indicating that the polymer effect is independent of known resistance mechanisms.

### In vivo intratumor efficacy studies:

Intratumor treatment of syngenic C57BL/6J mice bearing TRAMP-C2 tumor was conducted using NC₁₂K(KNC₁₂K)₃NH₂, an exemplary polymer according to the present embodiments. Isograft was induced by subcutaneous injection of 2.5 X 10⁶ cells. Mice (eight per group) were treated intratumorally on the days indicated by the arrows. The daily intratumoral treatment regime was conducted one week after cancer-cells implantation, when tumors were just palpable.

Briefly, the polymer's anti-tumor effect was examined in syngenic C57BL/6J mice grafted with tumorigenic TRAMP-C2 murine prostate cells (transgenic line of these mice). 0.1 ml of 2.5 X 10⁶ tumor cell suspended in cell medium without serum were inoculated subcutaneously into the left flank of 4-8 week-old mice. When volume of tumors reached 40-50 mm³, polymer injections of 50 µl (5 mg/kg) were performed intratumorally on a daily basis starting from day 8 through day 13, and then from day 20 through day 27. At day 28 mice were sacrificed and tumors were resected, weighted and photographed, and tumor size was measured by caliper.

Figures 14A-B present comparative plots showing the growth curves for each mouse when treated intratumorally with control PBS (Figure 14A) or 5 mg/kg of NC₁₂K(KNC₁₂K)₃NH₂ (Figure 14B). The insets in both figures focus on the first 14 days after cells implantation of the PBS treated mice (see insert in Figure 14A) and the polymer treated mice (see insert in Figure 14B).

Figure 15 presents two series of color photographs, showing tumors which were resected from syngenic C57BL/6J mice which were grafted with tumorigenic TRAMP-C2 murine prostate cells and then treated with PBS as control (left-side series) and NC₁₂K(KNC₁₂K)₃NH₂, and exemplary anticancerous polymer as presented herein, and showing the notable anticancerous effect of the polymer *in vivo.*

As can be seen in Figures 14A-B and Figure 15, NC₁₂K(KNC₁₂K)₃NH₂ significantly inhibited (P<0.5) tumor progression in most of treated mice, as indicated by the tumor volume measurements and confirmed post-mortem by tumor weights. Indeed, averages of extracted at day 28 tumors were 210±18 mg for control (PBS treated group) compared with 7±5 mg for the polymer treated group. As can further be seen in insets of Figure 14, the tumor completely disappeared in 7 out of 8 treated mice at day 14, six days after treatment onset. When treatment was stopped for the next six days, most tumors have resumed growth but were slowed down again upon polymer re-administration.

### Pharmacokinetic studies:

Pharmacokinetic studies were conducted by analysis of the blood and plasma of treated animals. Briefly, polymer solutions in PBS were injected intravenously (0.1 ml through the tail vein) and intraperitonealy (0.3 ml through peritoneal cavity) to C57BL/6J mice in a single dose of 100 µg/mouse. At the specified time periods following injection, mice (two per time point) were euthanized by CO₂ asphyxiation, their blood collected from the portal vein into heparinized tubes, then 100 µl of blood were mixed with 1 ml of extraction buffer (acetonitrile:formic acid (9:1 v/v) supplemented with 50 mM of ammonium formate) and incubated under shaking for 30 minutes. The remaining blood was centrifuged for 2 minutes at 6000 x g for plasma isolation. 100 µl of plasma were processed as described above for blood samples. After incubation, blood and plasma aliquots were vortexed, sonicated, centrifuged (2 minutes at 22,000 x g) and 150 µl of each sample were added to 150 µl of water containing 0.1 % trifluoroacetic acid for LC-MS analysis. A standard calibration curve was obtained after addition of known polymer concentrations to blood and plasma samples that followed identical treatment as above.

Figure 16 presents comparative plots, showing the concentrations of NC₁₂K(KNC₁₂K)₃NH₂, an exemplary polymer according to the present embodiments, as determined after 100 µg/mouse intraperitoneal administration (marked in black circles in Figure 16) and intravenous administration (marked in white circles in Figure 16) of the polymer in whole blood (Figure 16A) and in plasma (Figure 16B) after 30 minutes incubation at 25 °C in the extraction buffer (dashed line defines the limit of detection).

As can be seen in Figure 16, a single-dose intravenous administration of the anticancerous polymers presented herein was detectible in the bloodstream for about an hour, whereas after intraperitoneal administration the polymers rapidly reached the bloodstream and persisted for several hours.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims.

All publications, patents and patent applications mentioned in this specification are herein incorporated in their entirety by reference into the specification, to the same extent as if each individual publication, patent or patent application was specifically and individually indicated to be incorporated herein by reference. In addition, citation or identification of any reference in this application shall not be construed as an admission that such reference is available as prior art to the present invention.

## Claims

1. A polymer comprising a plurality of residues, wherein said plurality of residues comprises a plurality of amino acid residues and at least one hydrophobic moiety residue, whereas at least one of said at least one hydrophobic moiety residue is being covalently linked to at least two amino acid residues in said plurality of amino acid residues via an amine group of one amino acid residue and via a carboxyl of the other amino acid residue in said at least two amino acid residues, said polymer being a cyclic polymer, and is being identified for use in a method of treating cancer in a subject in need thereof.

2. A pharmaceutical composition comprising, as an active ingredient a polymer which comprises a plurality of residues, wherein said plurality of residues comprises a plurality of amino acid residues and at least one hydrophobic moiety residue, whereas at least one of said at least one hydrophobic moiety residue is being covalently linked to at least two amino acid residues in said plurality of amino acid residues via an amine group of one amino acid residue and via a carboxyl of the other amino acid residue in said at least two amino acid residues, said polymer being a cyclic polymer, the composition being packaged in a packaging material and identified in print, in or on said packaging material, for use in the treatment of cancer.

3. The polymer or composition of any one of claims 1 and 2, wherein said cancer is a multidrug resistant cancer.

4. The polymer or composition of any one of claims 1-3, wherein said hydrophobic moiety residue comprises at least one fatty acid residue.

5. The polymer or composition of claim 4, wherein said at least one hydrophobic moiety is an ω-amino-fatty acid residue.

6. The polymer or composition of claim 5, wherein each of said hydrophobic moieties if an ω-amino-fatty acid residue.

7. The polymer or composition of claim 6, wherein each of said ω-amino-fatty acid residues is independently selected from the group consisting of 4-amino-butyric acid residue, 6-amino-caproic acid residue, 8-amino-caprylic acid residue, 10-amino-capric acid residue, 12-amino-lauric acid residue, 14-amino-myristic acid residue, 16-amino-palmitic acid residue, 16-amino-palmitoleic acid residue, 18-amino-stearic acid residue, 18-amino-oleic acid residue, 18-amino-linoleic acid residue, 18-amino-linolenic acid residue and 20-amino-arachidonic acid residue.

8. The polymer or composition of claim 6, wherein each of said ω-amino-fatty acid residues is independently selected from the group consisting of 4-amino-butyric acid, 8-amino-caprylic acid and 12-amino-lauric acid.

9. The polymer or composition of any one of claims 1-8, wherein said plurality of amino acid residues substantially consists of positively charged amino acid residues.

10. The polymer or composition of claim 9, wherein each of said positively charged amino acid residues is independently selected from the group consisting of a lysine residue, a histidine residue, an ornithine residue and an arginine residue;

11. The polymer or composition of claim 9, wherein said positively charged amino acid residues are lysine residues.

12. The polymer or composition of any one of claims 1-11, wherein said polymer is selected from the group of the cyclic polymers presented in Table 4.

13. The polymer or composition of any one of claims 1-12, wherein said polymer has the general formula II: 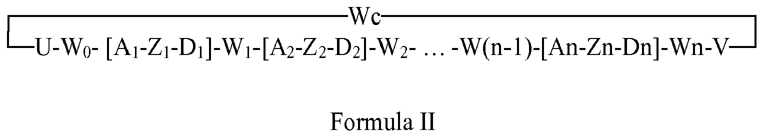 or a pharmaceutically acceptable salt thereof,
wherein:
n is an integer from 2 to 50;
A₁, A₂, ..., An are each independently an amino acid residue;
D₁, D₂, ..., Dn are each independently a hydrophobic moiety residue or absent, provided that at least one of said D₁, D₂, ..., Dn is said hydrophobic moiety residue;
Z₁, Z₂, ..., Zn and W₀, W₁, W₂, ..., Wn are each independently a linking moiety linking an amino acid residue and a hydrophobic moiety residue, or absent;
X and Y are each independently hydrogen, an amine, an amino acid residue, a hydrophobic moiety residue, has said general Formula I or absent;
W₀ is a linking moiety linking one of said A₁, Z₁ and D₁ to U, or absent;
Wn is a linking moiety linking one of said An, Zn and Dn to V, or absent;
U is selected from the group consisting of a first functional group, an amino acid residue having said first functional group, a hydrophobic moiety residue having said first functional group, and a linking moiety having said first functional group or absent;
V is selected from the group consisting of a second functional group, an amino acid residue having said second functional group, a hydrophobic moiety residue having said second functional group, and a linking moiety having said second functional group or absent; and
Wc is a cyclizing moiety.
